# EUROPEAN PATENT SPECIFICATION

(11) **EP 1 301 208 B1**
(45) Date of publication and mention of the grant of the patent: **03.02.2010**
(21) Application number: 01937864.5
(22) Date of filing: 06.06.2001
(51) Int. Cl.: A61K 47/36, A61P 15/18, A61P 37/04, A61K 47/48, A61K 39/39

(54) **Mannose as intranasal vaccine adjuvant**
Mannose als Adjuvant für intranasale Vakzine
Mannose comme adjuvant de vaccins intransal

(30) Priority: 06.06.2000 AU PQ797700
(43) Date of publication of application: 16.04.2003
(73) Proprietor: THE AUSTIN RESEARCH INSTITUTE, Heidelberg, Victoria 3084 (AU); The University of Melbourne, Parkville, Victoria 3052 (AU); Stambas, John, Footscray VIC 3011 (AU); Cheers, Christina, Victoria 3429 (AU)
(72) Inventor: McKENZIE, Ian, Farquhar, Campbell, Brunswick, VIC 3056 (AU); PIETERSZ, Geoffrey, Allan, Greensborough, VIC 3088 (AU); CHEERS, Christina, Sunbury, VIC 3429 (AU); STAMBAS, John, Footscray, VIC 3011 (AU); REYNOLDS, Eric Charles, North Balwyn, Victoria, 3104 (AU); O`BRIEN -SIMPSON, Neil Martin, Brunswick Victoria, 3056 (AU)
(74) Representative: Goulard, Sophie
(86) International application number: PCT/AU2001/000669
(87) International publication number: WO 2001/093912

(56) References cited:
- EP-A- 0 659 768
- WO-A-95/18145
- WO-A-98/28003
- WO-A-99/16455
- WO-A-99/17783
- WO-A-99/55715
- US-A- 5 597 807
- MCKENZIE IAN F C ET AL: "Oxidised mannan antigen conjugates preferentially stimulate T1 type immune responses" VETERINARY IMMUNOLOGY AND IMMUNOPATHOLOGY, vol. 63, no. 1-2, 15 May 1998 (1998-05-15), pages 185-190, XP002296500 ISSN: 0165-2427
- LETT E ET AL: "Mucosal immunogenicity of polysaccharides conjugated to a peptide or multiple-antigen peptide containing T- and B-cell epitopes." INFECTION AND IMMUNITY. JUL 1995, vol. 63, no. 7, July 1995 (1995-07), pages 2645-2651, XP002296499 ISSN: 0019-9567
- VAUGHAN HILARY A ET AL: "Induction of humoral and cellular responses in cynomolgus monkeys immunised with mannan-human MUC1 conjugates" VACCINE, vol. 17, no. 20-21, 4 June 1999 (1999-06-04), pages 2740-2752, XP002296501 ISSN: 0264-410X
- TODA S. ET AL: 'HIV-1-specific cell-mediated immune responses induced by DNA vaccination where enhanced by manna-coated liposomes and inhibited by anti-interferon-gamma antibody' IMMUNOLOGY vol. 92, 1997, pages 111 - 117, XP002977986
- SASAKI S. ET AL: 'Human immuno deficiency virus type-1 specific immune responses induced by DNA vaccination are greatly enhanced by mannan-coated di C14-amidine' EUROPEAN JOURNAL OF IMMUNOLOGY vol. 27, 1997, pages 3121 - 3129, XP000867270
- LEHNER T. ET AL: 'The Effect of Route of Immunization on Mucosal Immunity and Protection' THE JOURNAL OF INFECTIOUS DISEASES vol. 179, 1999, pages S489 - S492
- LETT E.: 'Immunogenicity of polysaccharides conjugated to peptides containing T- and B-cell epitopes' INFECT IMMUN vol. 62, no. 3, March 1994, pages 785 - 792
- APOSTOLOPOULOS V. ET AL: 'Cell-mediated immune responses to MUC1 fusion protein coupled to mannan' VACCINE vol. 14, no. 9, June 1996, pages 930 - 938, XP002097414

## Description

The present invention relates to a composition used in vaccination and to a vaccine, adapted for administration at a mucosal site.

### BACKGROUND OF THE INVENTION

Most infectious agents enter the body through mucosal membranes, and recent vaccine strategies have concentrated on the production of antibodies at these sites to block their entry. The stimulation of secretory immune responses, which includes mucosal IgA, the predominant antibody isotype in mucosal secretions with the capability to neutralise bacteria, bacterial products and viruses, is considered to be crucial to vaccine development [1-4]. Currently most vaccines delivered by injection are not efficient at inducing a mucosal response.

At present the oral polio vaccine remains the only successful large-scale vaccine that gives protection at the mucosal level. It is a live vaccine whose attenuation was achieved empirically with the attendant risks of back-mutation. In contrast, immunisation with defined protein antigens would have many advantages, including safety in immunosuppressed individuals. However, before such vaccines can be developed there is a need for adjuvants and delivery systems to overcome the problems associated with mucosal vaccination using defined protein antigens, including poor immunogenicity or the induction of tolerance.

Bacterial enterotoxins such as cholera toxin (CT) produced by *Vibrio cholerae*, and labile toxins from *Esherichia coli* have been co-administered with antigens and act as adjuvants with antigens [5,6]. However, these adjuvants pose health risks due to their toxic properties, and for the most part, cannot be used in human trials, and have limited usefulness for human vaccination. With regard to cholera toxin, Wiedermann, John-Schmid and Lindblad *et al* [7] showed that the tolerogenic or immunogenic properties for the B subunit of cholera toxin depended on the nature of the antigen/allergen to which it is coupled. Thus, in addition to its toxicity, CT suffers from a major disadvantage in that it may not be effective as an adjuvant, but rather, lead to tolerance.

In other studies, a genetically modified, detoxified form of the heat-labile toxin of enterotoxigenic *E. coli* (LT(RI92G)) was used intranasally in mice together with C. albicans (a fungus which causes "Thrush"), and protection against *C. albicans* infection was indicated [8].

Vaccines containing DNA encoding the antigen and low-viscosity carboxymethylcellulose sodium salt (CMCS-L) as a vehicle to carry the DNA to its site of action have been studied [9]. Lipids, such as monophosphoryl lipid A (CMPL) [10] which presumably aids in uptake of the antigen by the mucosal cell membrane, have also been used. However, the usefulness of these as adjuvants is unclear.

Adjuvants which have been administered with immunogens via the mucosa have therefore consisted of potentially dangerous bacterial or viral derivatives and display variable degrees of adjuvanticity.

The development of efficacious mucosal vaccines has further been hindered by incomplete understanding of mechanisms of pathogen transmission, and the immune responses specific to each pathogen. For example, development of a potent mucosal HIV vaccine has been affected by lack of understanding of mucosal HIV infection, and the immune responses that control the infection. There are studies which show that passive administration of IgG can confer protection against HIV infection at a mucosal site. However, the protection is limited, in that it appears unlikely that the protection is totally via neutralisation of the initial cell infection. It seems more likely that neutralisation at secondary lymphoid sites are important in controlling pathogen transmission via the mucosa [11]. Thus, many different approaches have been tried, to induce mucosal immunoglobin based immunity - like by immunising systematically and by immunising at a mucosal site. In most cases, the approaches have failed and are either toxic, or do not give rise to efficacious and practical immunoglobin based immune response.

Mannan, a polymannose or polysaccharide derived from the cell wall of yeast, when oxidised and conjugated to human Mucin 1 (an overexpressed cancer antigen) has been used in mice to induce immune responses [12, 13]. Intraperitoneal immunisation resulted in the induction of cellular immune responses as shown by production of CTLs and their precursors, Th1 cytokines IFN-γ and IL-12. Antibody production was usually low. In tests in more than 100 patients, mannan has not shown any obvious toxicity or autoimmunity [14].

Mannose receptors which bind mannan have so far been identified on macrophages and dendritic cells. Oxidised mannan has been shown to stimulate production of interleukin 12 in macrophages, and also to stimulate T-cells, and to cause rapid trafficking of antigens to the class I pathway to produce cytotoxic T-cells in mice [15].

Surprisingly the inventors have discovered that if mannan is administered via a mucosal site beneficial immunogenic effects are produced.

### SUMMARY OF THE INVENTION

The present invention is limited by the claims.

In a first aspect, the invention relates to the use of a composition comprising:
an antigen; and
a carbohydrate polymer comprising mannose, wherein said polymer
   a) comprises mannose monomers oxidized to produce aldehyde groups, and
   b) is covalently bonded to the antigen;
for the preparation of a vaccine for intranasal administration to generate an immune response to the antigen comprising a secretory immune response.

The immunisation preferably results in a humoral or cellular immune response. Preferably, it results in a humoral response. More preferably, it results in a humoral response wherein one or more of IgA, IgG, IgM and optionally, IgE antibody production is stimulated. In some instances, stimulation of IgE production may be beneficial, eg to immunise against worm infections.

In other instances, a reduction in total IgE production, or a reduction in the level of IgE relative to other antibody classes, can be beneficial, e.g. to prevent or reduce type I hypersensitivity or atopy, e.g.hayfever, asthma attacks or food and other allergies. IgE binding to its receptor and subsequent cross-linking with allergen is responsible for triggering immune responses underlying conditions such as asthma including atopic asthma, allergic rhinitis and atopic dermatitis, which are health problems of epidemic proportions. Thus, in one embodiment, the immune response is such that IgE production is reduced. In another embodiment, the IgE titre relative to one or more of IgA, IgG, IgM or subclasses of these is reduced. IgE production may be unchanged, or be substantially unchanged, whilst the production of one or more of the other antibodies is increased upon immunization with the composition.

Most preferably, IgA production is stimulated, and the titre of IgA at a selected mucosal area, and/or in the serum, is increased. In one embodiment, IgA production upon immunization is greater when compared with production of IgG, IgM and IgE. In a preferred embodiment, the immunisation selectively stimulates production of one or more of IgA, IgG and IgM over IgE. The immunoglobulins may include one or more of the subclasses within each class of antibody, for instance IgG2a and IgG1. Thus, in another preferred embodiment, immunization results in greater production of IgA relative to the increase in IgG1 and/or IgG2a production. The levels of antibody may be increased in serum or at mucosal sites, or both.

The subject immunised with the composition may be a human or an animal. For example the invention may be used to prevent and/or treat animal diseases. Thus, the antigen may be any pathogen known to cause diseases or infections in any animal species that include but are not limited to farm animals such as pigs, cattle, sheep, horses, and domestic animals and/or household pets such as cats and dogs, exotic animals such as zoo animals and feral animals. The antigen may also be an antigenic part of any of the pathogens. The types of infections or diseases that may be prevented or treated in accordance with the invention include but are not limited to those described in " Hagen and Bruner's Microbiology and Infectious Diseases of Domestic animals: with reference to etiology, epizootiology, pathogenisis, immunity, diagnosis and antimicrobiol susceptibility", WA Hagen, Comstock Pub. Associates, 1988.

The secretory immune response is preferably IgA immune response at one or more mucosal sites. Stimulation of secretory immune responses at these sites is particularly advantageous, as it can assist in neutralisation of pathogens or infectious agents upon their entry into the body through mucosal membranes.

The mucosal site may be a region or any area, from any mucosal surfaces, e.g. those lining the oral cavity or tissues, including the teeth and gingivae, those lining the gastrointestinal tract, or those lining the nasal passages and lungs, and the reproductive tract/tissues. The conjunctiva of the eyes also provides a suitable surface for administration of the composition. Examples of mucosal sites or surfaces include but are not limited to the respiratory tract such as the nasal region (e.g. the nose), the trachea, bronchi and the lungs, the buccal or oral tissues including the oral (e.g. the mouth and gingivae) and oro-pharyngeal cavities, the throat including the tonsils, the gastrointestinal tract (e.g. oesophagus, stomach, duodenum, small and large intestines, colon and rectum). An increase in antibody production in the male and female urinary and reproductive tracts is also contemplated and includes but is not limited to the bladder, ureter, urethra and associated tissues, the penis, the vulva/vagina and cervico-vaginal tissues, as well as the uterus and fallopian tubes.

Preferably, antibody production is increased in the respiratory tract such as the nasal region (e.g. the nose), the trachea, bronchi and the lungs, the buccal or oral tissues including the oral (e.g. the mouth and gingivae) and oro-pharyngeal cavities, the throat including the tonsils, the gastrointestinal tract (e.g. oesophagus, stomach, duodenum, small and large intestine, colon and rectum). An increase in antibody production in the male and female urinary and reproductive tracts is also contemplated and these include but is not limited to the bladder, ureter, urethra and associated tissues, the penis, the vulva/vagina and cervico-vaginal tissues, as well as the uterus and fallopian tubes. In a particularly preferred embodiment, the titre of IgA upon immunization is increased in a mucosal region selected from the group consisting of the lungs, nasal region, throat, gut, and male and female urinary and reproductive tracts. In another embodiment, the titre of one or more of IgA, IgG, IgM and, optionally, IgE is increased. In yet another embodiment, the level of one or more of IgA, IgG or IgM is increased to a greater degree than IgE. The immunoglobulins may include one or more sub-classes within each class of antibody.

In a further embodiment, the titre of IgA is increased upon immunization, to a greater degree than increases in IgG and IgM. Preferably, the increase in serum IgA upon immunization is greater than the increase in IgG 1 and/or IgG2a.

In a third aspect, the invention relates to a method of modulating an immune response at a mucosal site in a subject, comprising the step of administering a composition comprising:-
an antigen; and
a carbohydrate polymer comprising mannose to a mucosal site.

The immune response is preferably modulated so that antibody production, in response to administration of the composition, is selected from the group consisting of IgA production, IgG production, IgM production and IgE production. The immune response may also be modulated by selectively inducing a desired type of antibody response, for example, by increasing its level of production so that a greater amount is produced when compared with another class or subclass of antibody. In one embodiment, the immune response is modulated by inducing an increase in production of one or more of IgA, IgG and IgM relative to that of IgE. More preferably, the immune response is modulated by increasing production of IgA relative to that of IgG, IgM and/or IgE. Most preferably, the immune response is modulated such that IgA production is increased relative to the production of IgG, particularly IgG1 and/or IgG2a. The immune response may also be modulated by inducing production of antibodies whose presence in turn modulates response to other classes of antibodies. In a preferred embodiment, the immune response is modulated such that production of one or more of IgA, IgG1, IgG2a or IgM attenuates or inhibits the action of IgE. In another embodiment, the production of one type of antibody stimulates the action of another class of antibody.

Alternatively the immune response is preferably modulated so that mediators of cellular immunity are stimulated, such as Th1 and/or Th2.

In yet another embodiment, the mucosal or secretory immune response is stronger than the systemic immune response. In a preferred embodiment, the secretory immune response is an IgA response, and the systemic immune response is IgG response.

The antigen which evokes the immune response may include and is not limited to all or immunogenic portions of pollens, allergens especially those that induce asthma, bacteria, viruses, yeasts, fungi, protozoa and other microorganisms, or pathogens of human, animal or plant origin. Derivatives of these antigens are also contemplated and may be homologues, analogues, conjugates or salts of such entities.

In a preferred embodiment, the antigen is selected from the group consisting of influenza virus, haemagglutinin of influenza, Porphyromonas gingivalis (which causes gum disease and cardiovascular disease), proteinase and adhesin epitopes of Porphyromona gingivalis, Helicobacter pylori, urease of Helicobacter pylori, rotavirus, recombinant outer capsid proteins of rotavirus, HIV, gp120 of HIV, RSV, surface proteins of RSV, antigens from microorganisms which cause venereal disease and ovalbumin peptides.

Other antigens or antigenic portions or derivatives thereof may also be used in accordance with the invention. These include but are not limited to *Listeria monocytogenes, Mycobacterium tuberculosis, BCG, Mycobacterium avium, M avium hsp65,* influenza nucleoprotein, Respiratory Syncyticial virus (RSV) F or G proteins, HIV, *Candida especially Candida albicans* and *Chlamydia trachomatis* or outer membrane proteins thereof, *Neisseria meningitidis* class 1 outer protein, Herpes simplex virus type I glycoprotein G or gp D or CP27, Human Papilloma Virus antigen E7, Porphyromonas gingivalis Cpx, Murray valley encephalitis virus E glycoprotein, antigenic portions thereof and derivatives thereof. Antigens selected from the group consisting of those pathogens which cause the common cold, those which cause chlamydia, from *Streptococcus,* from *Staphylococcus* may also be used in accordance with the invention. Antigens corresponding to, or derived from, the organisms disclosed in "Medical Biology, a Guide to Microbial Infections: Pathogenesis, Immunity, Laboratory Diagnosis and Control", D Greenwood, R Slack and J Peutherer (eds), 15th edition, Churchill Livingston, Edinburgh, 1997, are also contemplated.

In another embodiment of the invention, the antigen is selected from the group consisting of *Listeria monocytogenes, Mycobacterium tuberculosis, Chlamydia trachomatis, Chlamydia pneumoniae, outer membrane proteins thereof,* antigenic portions thereof, derivatives thereof, and synthetic peptides based on epitopes from these organisms. In one embodiment, the antigen is selected from the group comprising thel9kDa lipoprotein of *M. tuberculosis,* hsp-65 of *M. avium,* VP5 ofrotavirus, E7 of HPV and Cpx of P. gingivalis.

A selected antigen may form part of a fusion protein in order to facilitate expression and purification on production of the fusion protein in recombinant host cells. The non-antigen portion of the fusion protein would generally represent the N-terminal region of the fusion polypeptide with the carboxy terminal sequences comprising antigen sequences. Fusion proteins may be selected from glutathione-S-transferase, β-galactosidase, or any other protein or part thereof, particularly those which enable affinity purification utilising the binding or other affinity characteristics of the protein to purify the resultant fusion protein. The protein may also be fused to the C-terminal or N-terminal of the carrier protein. The nature of the fusion protein will depend upon the vector system in which fusion proteins are produced. An example of a bacterial expression vector is pGEX which on subcloning of a gene of interest into this vector produces a fusion protein consisting of glutathione-S-transferase with the protein of interest. Examples of other vector systems which give rise to fusion proteins with a protein of interest are described in Sambrook et al, "Molecular Cloning: A Laboratory Manual", Cold Spring Harbor Press, Cold Spring Harbor, USA, 1989.

Alternatively synthetic peptides or epitopes, optionally coupled to a protein carrier may be used in the invention. Synthetic peptides or epitopes may be produced in accordance with standard methods.

The carbohydrate polymer comprising mannose is most preferably a carbohydrate polymer containing mannan. In a preferred embodiment the antigen is conjugated to oxidised mannan in a similar manner to that described in WO 95/18145. The mannan is preferably isolated from cell wall of yeast, and may be oxidised using reagents such as sodium periodate to produce a polyaldehyde which is then directly reacted with a selected antigen. Reduced mannan may also be used, and a composition containing this may be prepared by adding sodium borohydride to an oxidised mannan-antigen conjugate.

In one embodiment polysaccharide chains may be first activated with cyanogen bromide and the activated polysaccharide then reacted with a diamine, followed by conjugation to the antigen to form a conjugate which may optionally then be oxidized. The carbohydrate and polypeptide may be derivatized with bifunctional agents in order to cross-link the carbohydrate and polypeptide. Commonly used cross-linking agents include 1,1-bis (diazoacetyl)-2-phenylethane, glutaraldehyde, N-hydroxysuccinimide esters, for example, esters with 4-azidosalicyclic acid, homobifunctional imidoesters including disuccinimidyl esters such as 3,3'-dithiobis(succinimidyl-propionate), and bi functional maleimides such as bis-N-maleimido-1, 8-octane. Derivatizing agents such as methyl-3-[(p-azido-phenyl)dithio] propioimidate yield photactivitable intermediates which are capable of forming cross-links in the presence of light. Oxidised carbohydrates may be reacted with hydrazine derivatives of antigens to give a conjugate. Alternatively, carbohydrates may be reacted with reagents such as carbonyl diimidazole, which after oxidation gives the desired conjugate.

The coupling of antigens to carbohydrate involves converting any or all of the functional groups on the carbohydrate to reactive groups and thereafter reacting the reactive groups on the carbohydrate with reactive groups on the polypeptide. Carbohydrate polymers are replete with hydroxide groups, and in some instances, carboxyl groups (such as in iurodinate), ester groups (such as methylgalacturonate) and the like. These groups may be activated according to standard chemical procedures. For example, hydroxyl groups may be reacted with hydrogen halides, such as hydrogen iodide, hydrogen bromide and hydrogen chloride to give the reactive halogenated polysaccharide. Hydroxy groups may be activated with phosphorous trihalides, active metals (such as sodium ethoxide, aluminium isopropoxide and potassium tert-butoxide), or esterified (with groups such as tosyl chloride or acetic acid) to form reactive groups which can be then be reacted with reactive groups on the polypeptide to form one or more bonds. Other functional groups on carbohydrates apart from hydroxyl groups may be activated to give reactive groups according to well known procedures in the art.

Without wishing to be bound by any proposed mechanism for the observed advantages of the invention, it is thought that the mannan not only acts as an adjuvant, but is also a potent mucosal adjuvant by virtue of increased or efficient uptake via the mucosa.

The mucosa is as described above, and includes the mucosal surfaces of the respiratory tract such as the nasal region and the lungs, the GI tract such as the buccal or oral and oro-pharyngeal cavities, throat, tonsils and gut, the rectal tissues, and the male and female urinary and reproductive tracts including the cervico-vaginal tissues.

In a further aspect, the invention provides a composition adapted to be administered at a mucosal site thereby to generate an immune response, the composition comprising an antigen and a carbohydrate polymer comprising mannose. Preferably, the antigen, carbohydrate polymer and immune response are as described above. These antigens and carbohydrates are also preferably treated in the manner described above to form the composition.

The composition is preferably formulated for administration at a mucosal site by inhalation of the composition which is sprayed into the nasal region.

The composition is most preferably used to vaccinate a subject in need thereof via a mucosal site. The immune response is preferably a response as described above.

The composition or vaccine in accordance with the invention is administered via a mucosal site to human or animal patients to protect against various disease states including diseases or infection of the eyes such as trachoma or conjunctivitis, listeriosis, tuberculosis, influenza, colds, respiratory diseases, sexually transmitted diseases or infections by viruses, bacteria, fungi, protozoa, or other microorganisms or pathogens.

Administration or vaccination may be as described above, or may be a single or multiple event, or may be part of a prime-boost protocol, a combination of these, or each of these with other, conventional methods of vaccination. The prime-boost protocol may, for example, comprise priming by intramuscular administration, and boosting by intranasal administration. The prime-boost may, in addition to the composition of the invention, include DNA, viruses (eg vaccinia) or other immunogenic peptides or molecules, including the antigens described above. One or both of the priming and boosting composition may include the composition of the invention, namely, an antigen and a carbohydrate polymer comprising mannose. Thus, in a prime-boost protocol, one of the events may include the composition in accordance with the invention, while the other may omit the carbohydrate polymer containing mannose.

By way of example, 1µg to 10,000µg/kg may be administered to a subject, preferably 5µg to 5000µg/kg, more preferably 8-1000µg/kg and most preferably 400-600µg/kg. Even more preferably a dose of 100-200µg/kg is contemplated particularly for humans.

The compounds or vaccines of the invention may also be administered to subjects in conjunction with other immune regulators that lend themselves to efficacious administration via the mucosa.

Other adjuvants, pharmaceutically acceptable carriers, diluents or auxiliaries which may enhance the immunogenicity or effectiveness of the composition or vaccine of the invention may also be included therein, or be co-administered therewith. For instance, lipofectamine may be co-administered with the composition or vaccine of the invention.

The composition or vaccine may be formulated in accordance with the manner in which it is to be administered. For example, it may be formulated for inclusion in a spray-container, aerosol can or nebuliser for administration by inhalation. Or it may be formulated as a solution or liquid to be added dropwise or as droplets to a mucosal site, e.g. the oral cavity or throat for absorption. Nose-drop formulations, liposomal formulations, slow-release formulations, capsules or devices, or formulations comprising DNA are also contemplated.

The invention may be used to protect or treat animal and/or human subjects. Thus, in one embodiment, the invention provides a method of vaccinating a subject against *Chlamydia* comprising the step of administering a composition as described above. Preferably the antigen is outer membrane protein of C. *trachomatis* and the method is used in the treatment or prophylaxis of trachoma.

In another embodiment, the invention provides a method of immunising a subject with BCG, comprising the step of administering a composition as described above, wherein the antigen is BCG, or purified or recombinant preparations thereof. In a preferred embodiment, the subject is an animal such as a possum.

In a further aspect there is provided a composition comprising:
an antigen; and
a carbohydrate polymer comprising mannose to a mucosal site.

In one embodiment, administration of the composition in accordance with the above method results in production of antibody against sperm thereby to prevent fertilisation of an egg. The antibody is preferably IgA.

In another embodiment, the composition induces production of antibodies against the zona pellucida thereby to prevent maturation of an embryo. The antibody is preferably IgA. Such methods may be used in controlling fertility of subjects such as animals that kill native wild life, e.g. foxes, or animals that destroy natural vegetation, or that compete with livestock for food, e.g. rabbits. Populations of animals that are a reservoir for undesirable diseases, e.g. possums, which carry or act as a reservoir for tuberculosis, may also be controlled in accordance with the method of the invention.

The present invention is advantageous in that it stimulates an effective immune response in a non-invasive manner. For example, animals such as native species in danger of extinction due to diseases may be vaccinated by administering nasal droplets of an immunogenic composition in accordance with the invention. Non-invasive administration could also be achieved by use of aerosols or nebulisers, and are also likely to increase compliance in humans, particularly children.

The invention also extends to use of the composition described above in the manufacture of a medicament for modulating an immune response in a subject and use of the composition in the manufacture of a vaccine suitable for administration to a mucosal site.

### DESCRIPTION OF THE INVENTION

The scope of the present invention is defined by the claims.

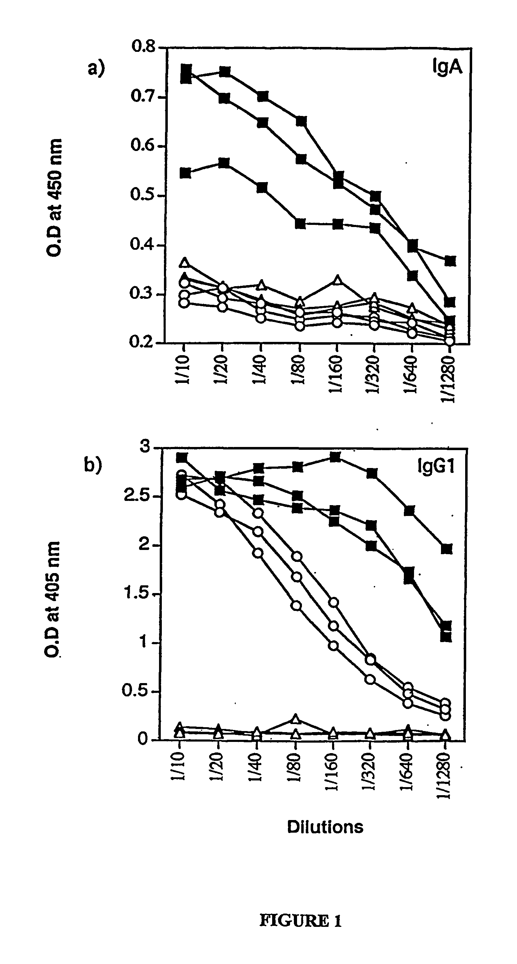

Preferred embodiments of the invention will now be described with reference to the following, non-limiting examples, in which:-
Figure 1 shows the results of intranasal versus intraperitoneal immunisation: (CBAxBALB/c) F1 mice were immunised on days 0, 10, 17 with 12µg of M-LLOFP intranasally or intraperitoneally, and serum was obtained from individual mice on day 24. Antigen specific IgA (Figure 1a) and IgG 1 (Figure 16) were detected by ELISA and optical density was measured at 450 nm. Closed squares (■) indicate intranasally immunised mice, open circles (○) intraperitoneally immunised mice and open triangles (△) the unimmunised controls. Results for individual mice are shown.

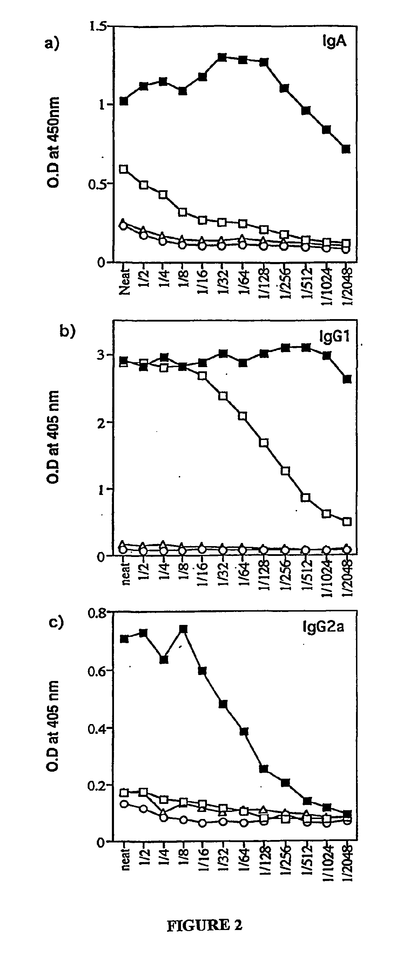

Figure 2 shows subclasses of antibody in the serum of M-LLO.FP immunised mice. (CBAxBALB/c) mice were immunised intranasally with 12µg of M-LLO.FP or unconjugated LLO.FP on day 0, 10 and 17 and serum from 5 individual mice obtained on day 24. Antigen specific IgA (a), IgG1 (b) and IgG2a (c) was detected by ELISA. Individual titres are shown as dot plots, with corresponding symbols for different antibody classes from the same mouse. In addition, titres were converted to geometric means (heavy bar) and standard deviations (vertical bar) for display. Differences between groups receiving M-LLO.FP or unconjugated LLO.FP were significant for all classes of antibody.

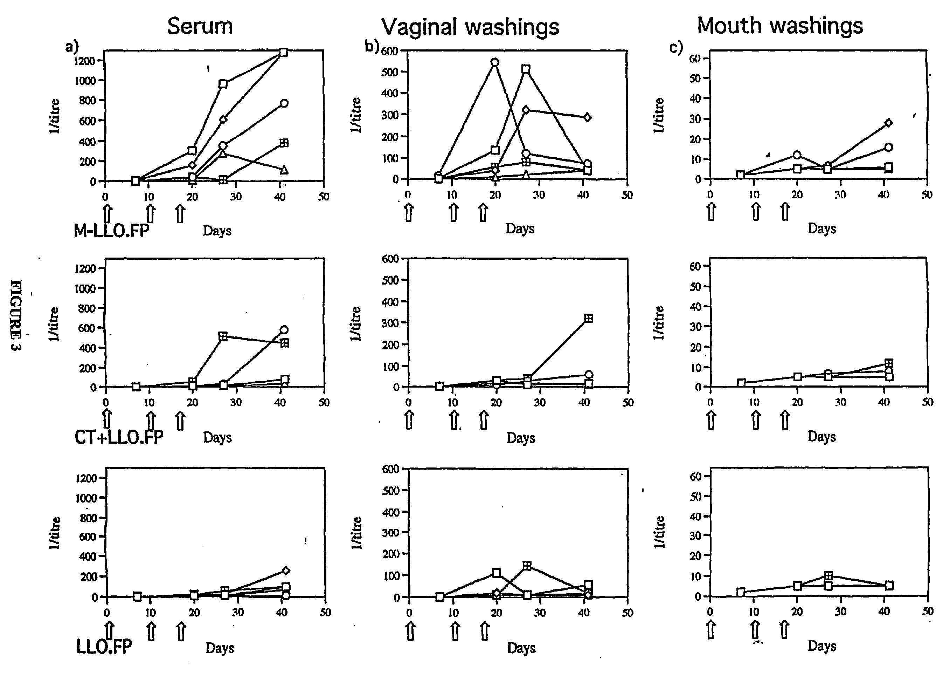

Figure 3 shows the levels of antigen specific IgA in the serum and distant mucosal sites over a 41 day period: (CBAxBALB/c) F1 mice were immunised on day 0,10 and 17 with 12µg of M-LLOFP, with 1µg of CT mixed with 12µg of LLOFP or with 12µg of LLOFP alone. Serum (a), vaginal washings (b), and mouth washings (c) were collected from 5 individual mice on days 7, 20, 27 and 41. Antigen specific IgA titres were determined by ELISA and the progress in individual mice shown.

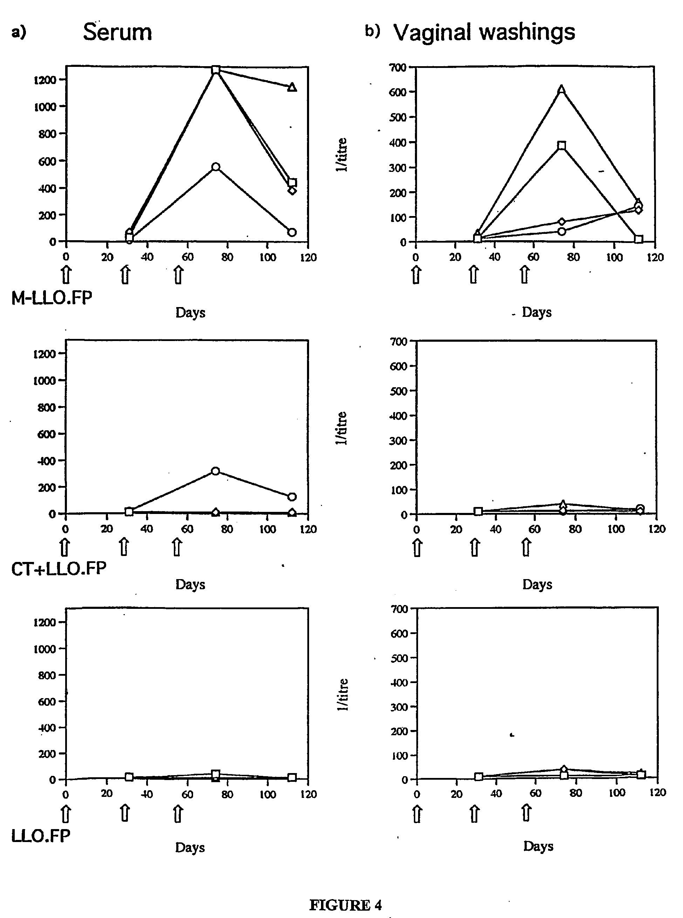

Figure 4 shows the antigen specific IgA at distant mucosal sites over a 112 day period: (CBAxBALB/c) F1 mice were immunised on day 0, 28 and 56 with 12µg of M-LLOFP, with 1µg of CT mixed with 12µg of LLOFP or with 12µg of LLOFP alone. Serum (a) and, vaginal washings (b) were collected from 4 individual mice on day 31, 74,and 112. Antigen specific IgA titres were determined by ELISA and the progress in individual mice shown.

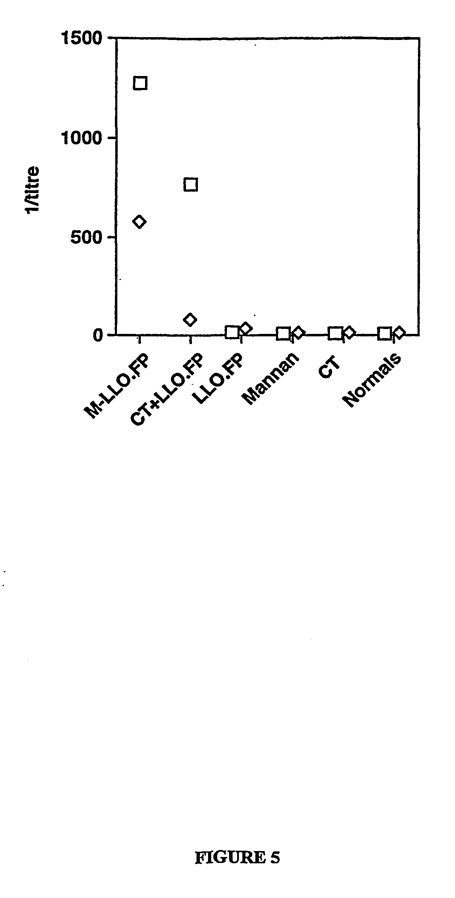

Figure 5 shows the antigen specific IgA in the tears and in the lung washings. (CBAxBALB/c) F1 mice were immunised on day 0, 10 and 17 with 12µg of M-LLO.FP or with 12µg of LLO.FP alone. Mice were anaesthetised on day 24 of the experiment and tears (1µl) collected from 5 mice per group (a). Mice were euthanased on day 35 of the experiment and lung washings (0.5ml) collected (b). Antigen specific IgA titres were determined by ELISA and individual titres are shown as dot plots. In addition, titres were converted to geometric means (heavy bar) and standard deviations (vertical bar) for display. Differences between groups receiving M-LLO.FP or unconjugated LLO.FP were significant (p<0.01) in tear samples and (p<0.05) for lung washings.

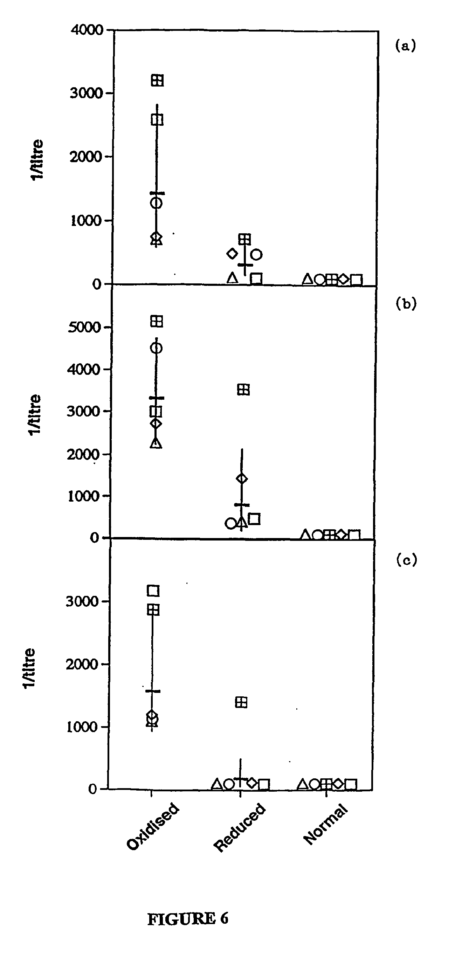

Figure 6 shows the effects of the M-LLOFP conjugate when in the oxidised form: (CBAxBALB/c) mice were immunised intranasally with 12µg of M-LLOFP in the oxidised or reduced form on day 0, 10 and 17. Serum from 5 individual mice was collected on day 24 and antigen specific IgA (a), IgG1 (b) and IgG2a (c) was detected by ELISA. Individual titres are shown as dot plots, with corresponding symbols for different antibody classes from the same mouse. In addition, titres were converted to log₁₀ and geometric means (heavy bar) and standard deviations (vertical bar) were derived and converted back to arithmetic numbers for display. Differences between groups receiving oxidised or reduced M- LLO.FP were significant (p<0.02) for all classes of antibody (Student's *t* test).

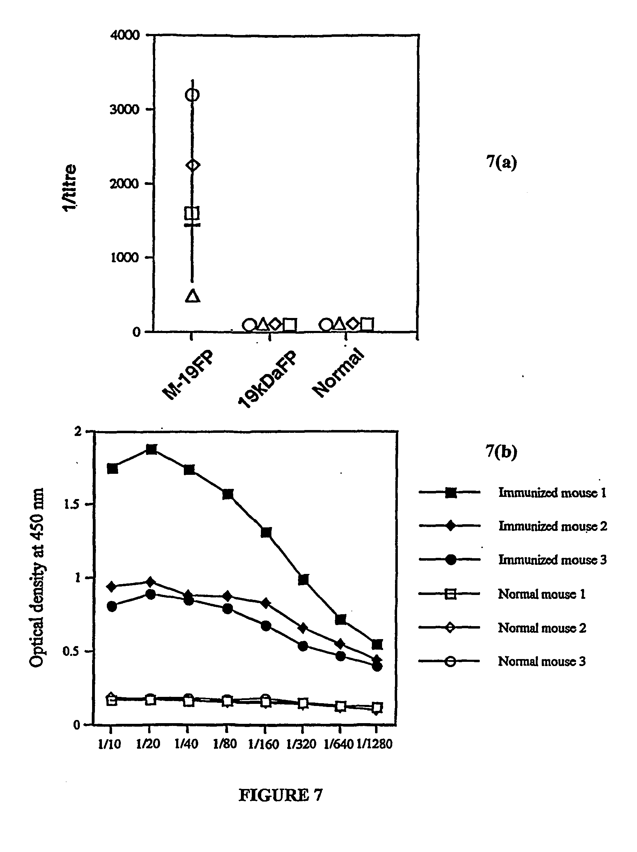

Figure 7(a) shows the effects of oxidised mannan as adjuvant for mycobacterial 19 kDa FP: Groups of 4 C57/B.10 mice were immunised intranasally with 12µg of M-19FP or 12µg of 19 kDaFP on day 0,10 and 17. Serum was collected on day 24 and antigen specific IgA determined by ELISA. Individual titres are shown as dot plots. In addition, titres were converted to log₁₀ and geometric means (heavy bar) and standard deviations (vertical bar) were derived and converted back to arithmetic numbers for display. Differences between groups receiving M-19FP and 19FP alone were significant by Student's *t* test (p<0.02).
Figure 7(b) shows ELISA readings for IgA titres for mice immunised with hsp-65 - mannan conjugate.

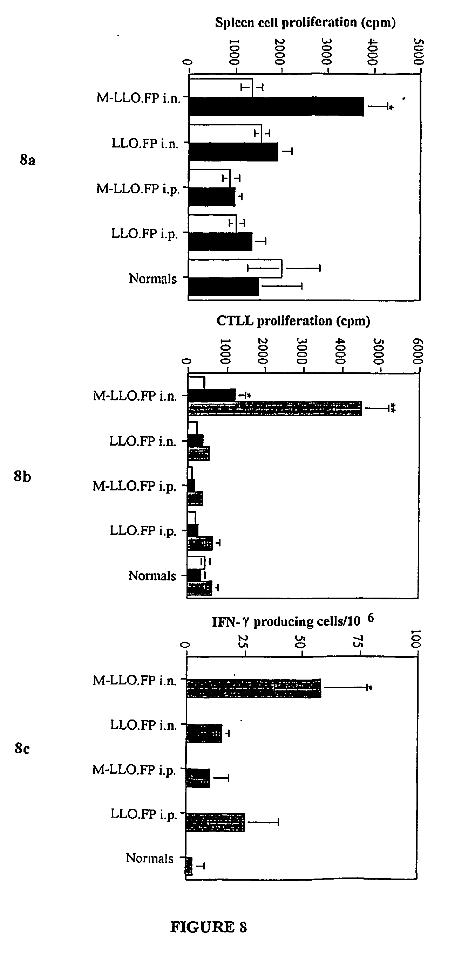

Figure 8. Comparison of intranasal and intraperitoneal immunisation with M-LLO.FP. (CBAxBALB/c) F1 mice (4 per group) were immunised with 12µg of M-LLO.FP i.n. or i.p. or unconjugated control on day 0, 10 and 17 and mice sacrificed on day 24. (a) Proliferative response: Spleen cells from all groups were cultured in the presence of antigen (45µg/ml LLO₍₂₁₅₋₂₂₆₎ (■) or without stimulus (%). Data represent the means and standard deviations of triplicate cultures. * p<0.002 compared with M-LLO.FP i.p. (b) IL-2 production: Spleen cells from all groups were cultured in the presence of antigen (45µg/ml LLO₍₂₁₅₋₂₂₆₎ (■) or LLO.FP (□)) or without stimulus (%). Data represent the means and standard deviations of triplicate cultures. * p<0.01 ** p<0.002 compared M-LLO.FP i.p.. (c) IFN-γ producing cells: Spleen cells from all groups were cultured in the presence of antigen (45µg/ml of LLO.FP (■)) or without stimulus (%). Data represent the means and standard deviations of triplicate cultures. * p<0.05 compared with M-LLO.FP i.p.

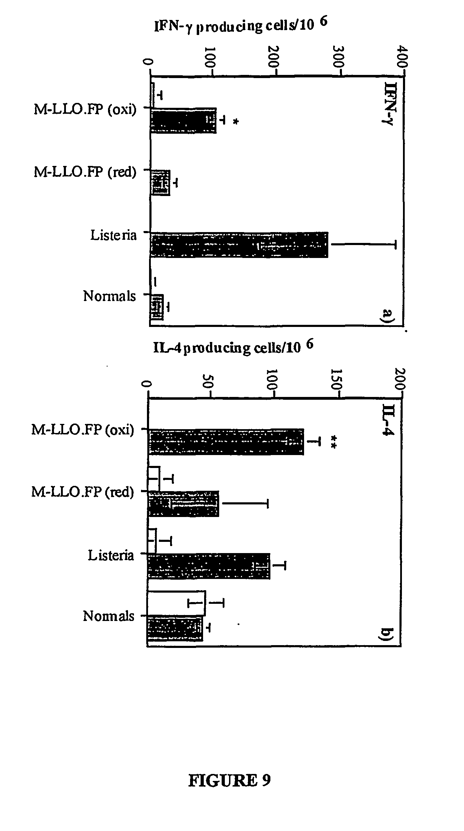

Figure 9. IFN-γ and IL-4 production following i.n. immunisation with oxidised or reduced M-LLO.FP. (CBAxBALB/c) F1 mice (5 per group) were immunised i.n. with 12µg of M-LLO.FP (oxidised) or with M-LLO.FP (reduced) on day 0, 10 and 17 or infected i.v. with 1x10³ Listeria on day 17. Mice were sacrificed on day 24. Spleen cells from all groups were cultured in the presence of antigen (45µg/ml of LLO.FP (■)) or without stimulus (%) and the frequency of (a) IFN-γ and (b) IL-4 producing cells determined. Data represent the means and standard deviations of triplicate cultures. *p<0.02, **p<0.05 compared with the reduced group for IFN-γ and IL-4 respectively. Note different scales.

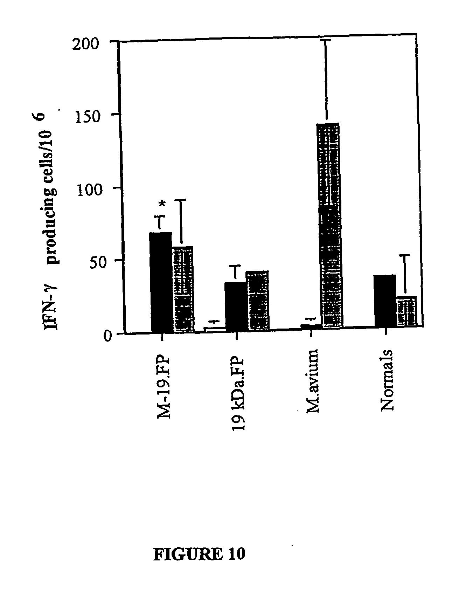

Figure 10. IFN-γ production following i.n. immunisation with M-19.FP. C57BL/10 mice (4 per group) were immunised i.n. with 12 µg of M-19.FP or unconjugated control on day 0, 10 and 17 or infected i.n. with *M. avium* 6 weeks prior to immunisation. Mice were sacrificed on day 24 and spleen cells from all groups cultured in the presence of antigen (■) (19 kDa.FP 23 µg/ml), 10⁷ live *M. avium* (□) or without stimulus (%). Data represent the means and standard deviations of triplicate wells. * p<0.05 compared with the 19.kDa.FP group.

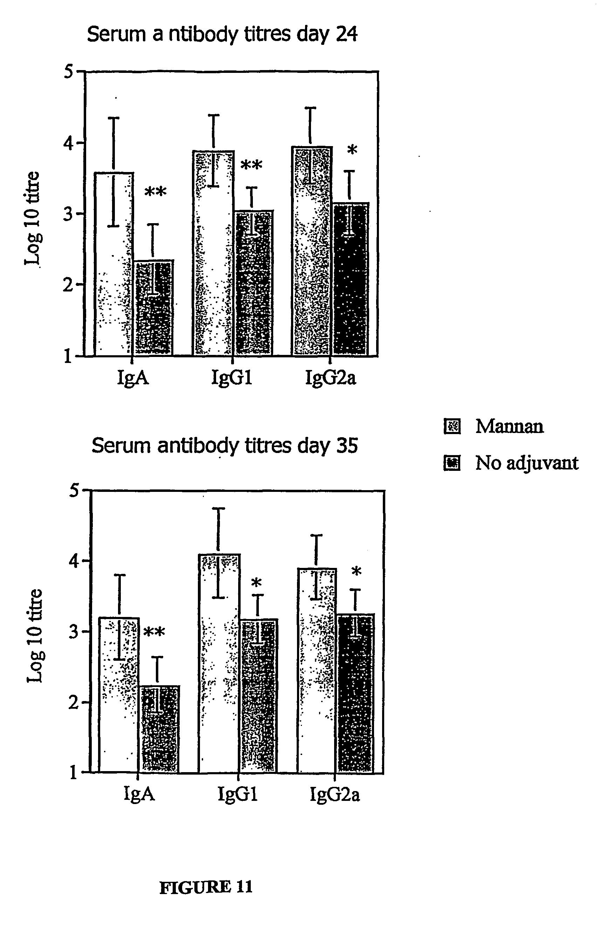

Figure 11: Antibody titres in serum following immunisation with VP5₂₄₆₋₂₇₄FP with and without mannan adjuvant: Mice were immunised intranasally with 6µg VP5₂₄₆₋₂₇₄ -mannan of VP5₂₄₆₋₂₇₄ alone on days 0,10 and 17, and bled from the tail vein on days 24 and 35. Titres are expressed as log 10 geometric means plus or minus standard deviation for groups of 5 mice. Differences p<0.05 are considered significant. **p<0.02, *p<0.05.

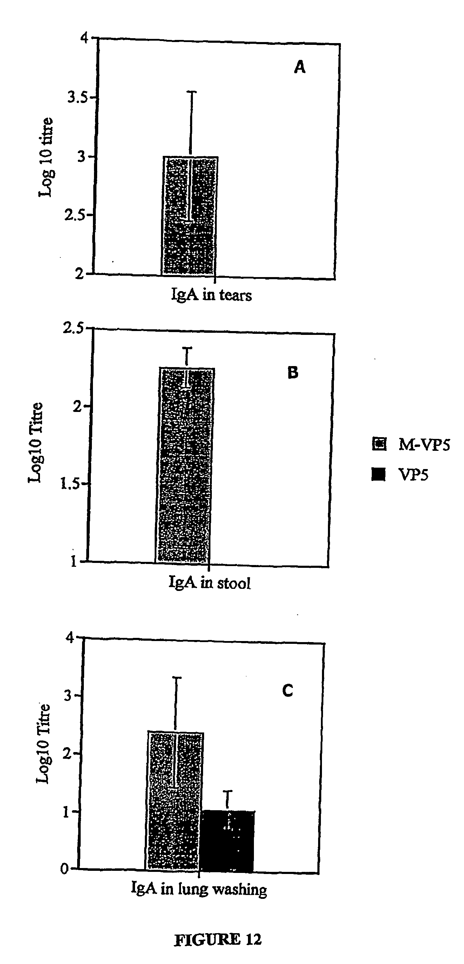

Figure 12: IgA titres on mucosal surfaces following immunisation with VP5₂₄₆₋₂₇₄FP with and without mannan adjuvant: Mice were immunised intranasally with 6µg VP5₂₄₆₋₂₇₄ -mannan of VP5₂₄₆₋₂₇₄ alone on days 0,10 and 17. Titres are expressed as log 10 geometric means plus or minus standard deviation for groups of 5 mice.
   (A). On day 24 tears were collected as described and IgA antibody assayed by ELISA. Note limit of detection was 1/100 and no antibody was detected in mice receiving antigen alone.
   (B). On day 34 stools were collected, homogenised in 10x volume of PBS and the extracted IgA antibody assayed by ELISA. Note limit of detection was 1/10 and no antibody was detected in mice receiving antigen alone.
   (C) On day 35 the mice were sacrificed and lung washings collected. Significant difference in titres, p<0.02.

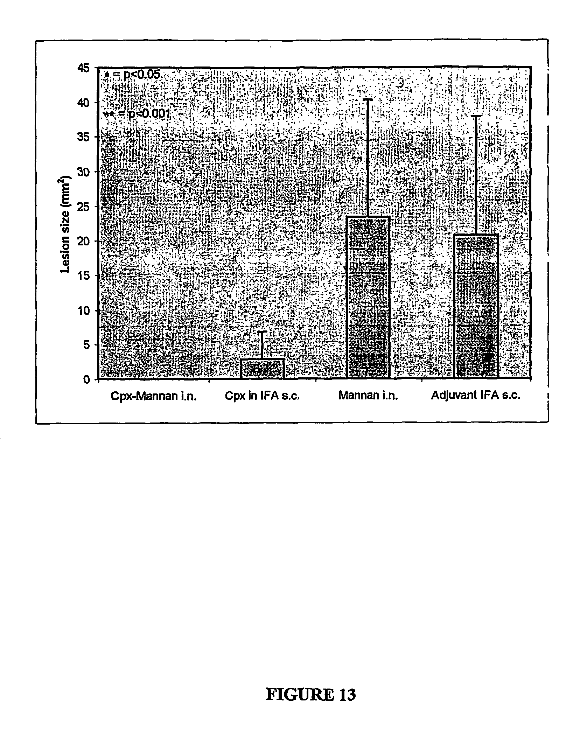

Figure 13. Protection against Porphyromonas gingivalis lesions: Groups of 5 C57B1/10 mice were immunised with Cpx-mannan given intranasally (Figure 13(a)) or Cpx in incomplete Freund's adjuvant (IFA) subcutaneously (Figure 13(b)). Control mice received the respective adjuvants alone. Mice given Cpx-mannan intranasally showed no lesions - complete protection (p<0.001), while those given Cpx in IFA showed incomplete protection (p<0.05).

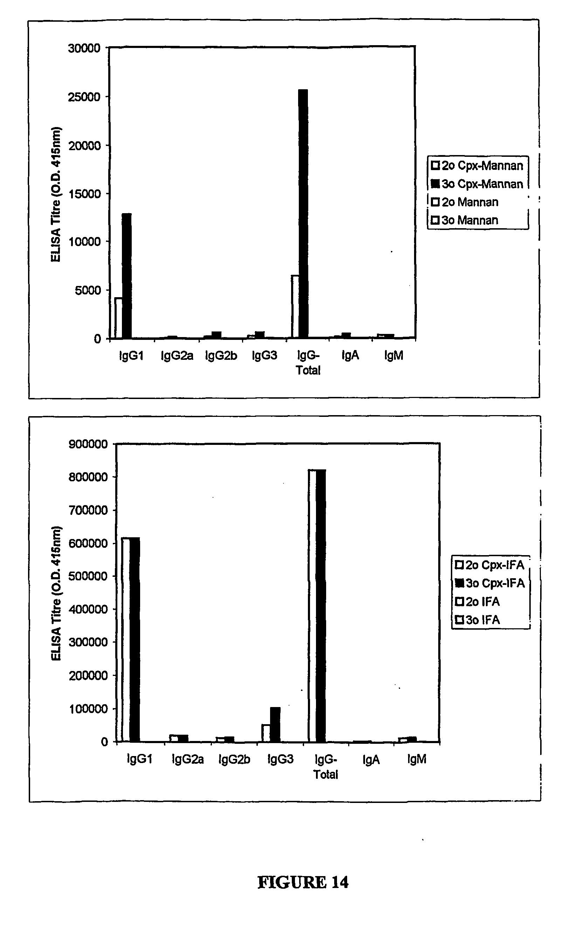

Figure 14. Serum antibody titres: Groups of 5 C57B1/10 mice were immunised with Cpx-mannan given intranasally or Cpx in incomplete Freund's adjuvants (IFA) subcutaneously. Control mice received the respective adjuvants alone. The mice were bled from the retro-orbital plexus after 2 or three doses and serum antibody assayed by ELISA. Titres on pooled sera are shown.

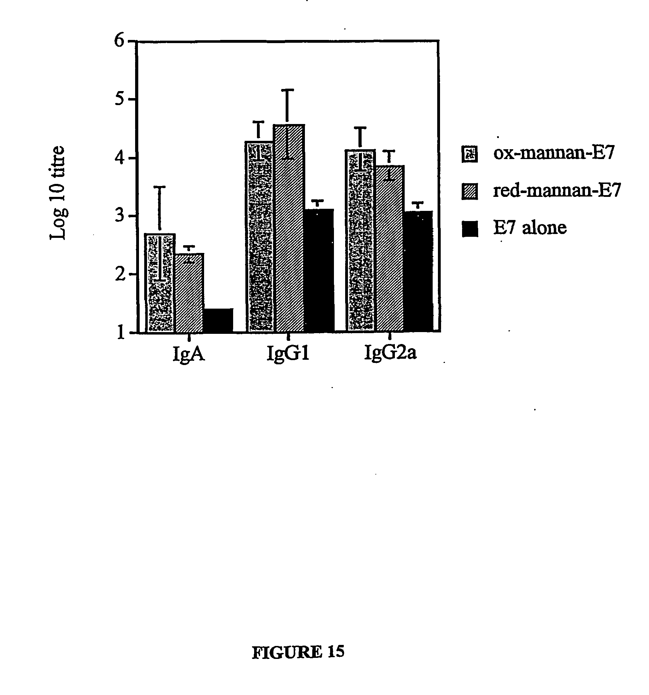

Figure 15. Antibody titres in serum following immunisation with E7.FP coupled to mannan under oxidising or reducing conditions or without mannan adjuvant (E7 alone): Mice were immunised intranasally with 12µg antigen (with or without mannan) on days 0, 10 and 17, and bled from the tail vein on days 24. Titres are expressed as log 10 geometric means plus or minus standard deviation for groups of 5 mice. Titres of all classes of antibody in groups immunised E7.FP plus either oxidised or reduced mannan were significantly different from the group receiving E7.FP, alone p<0.01.

### Example 1: Materials and General Methods

Mice: Female 6-8 week old (CBAxBALB/c)F1 and C57B1/10 mice were bred and maintained under conventional but infection-free conditions in the animal house of the Department of Microbiology and Immunology, University of Melbourne.

Production of antigens: A p. bluescript plasmid containing the gene for Listeriolysin O (LLO) from *Listeria monocytogenes* (without its leader sequence) was obtained from Richard Strugnell (University of Melbourne, Australia) and subcloned into the *Escherichia coli* expression vector pGEX-2T [16] in the correct reading frame and orientation. The expression of an 84 kDa LLO glutathione-s-transferase (GST) fusion protein (LLO.FP) was induced with 0.1 mM IPTG (Sigma Chemical Co., MO, USA) at 37°C for 5 hours. Bacteria were collected by centrifugation (1,500 x g) for 5 minutes, washed and lysed by sonication. The pellet was collected after centrifugation and solubilised in 8M urea (Eastman Kodak Co., Rochester, NY, USA) overnight at 4°C. After further centrifugation (26,000 x g) for 15 minutes the supernatant was dialysed in 0.01 M Tris (Sigma Chemical Co., MO, USA) pH 8.0 /1M urea and the BIOCAD perfusion chromatography system (Perceptive Biosystems, Framingham, MA, USA) used to purify the protein by anion exchange.

The 19 kDa lipoprotein from *Mycobacterium tuberculosis* was produced using a recombinant 19 kDa-plasmid construct was obtained from Professor Douglas Young (Imperial College School of Medicine, London, U.K) and the expression of a 19 kDa HIS tag fusion protein (19FP) induced with 0.1 mM IPTG at 37°C for 5 hours. Bacteria were collected by centrifugation (1,500 x g), washed and lysed by sonication. The supernatant was collected after further centrifugation (26,000 x g) for 15 minutes and dialysed in 0.01M Tris pH 8.0/ 300mM NaCl/ 20 mM imidazole and purified under native conditions on a Nickel chelate column (Qiagen, Hilden, Germany) using the BIOCAD perfusion chromatography system.

Rotavirus VP5 antigen was produced recombinantly using the gene for the fragment of VP5 from amino acid 248-475 (VP5₂₄₆₋₂₇₄) [17]. This gene fragment was cloned into the pGEX plasmid to create a fusion protein with glutathione S transferase. The recombinant E. coli was grown overnight at 37°C in LB-ampicillin medium and subcultured at 1:50 dilution into fresh LB-ampicillin medium. After incubation of the bacteria at 20°C until OD₆₀₀= 1.4-1.6 (about 12-16 hours), expression of the fusion protein was induced with 0.01mM IPTG for 6-8 hours at 20°C. Bacteria were harvested and washed before sonication in PBS for 15 seconds each. Centrifugation followed by passage through a 0.45µm filter was used to collect soluble protein. The GST fusion protein was purified using Glutathione Beads, according to the manufacturers' instructions (Sigma, Missouri, USA). The eluted GST-VP5 was passed through the 0.45µm filter to remove traces of beads and the protein dialysed against pH9 bicarbonate buffer.

The antigen, hsp65 of *Mycobacterium avium* was produced recombinantly according to standard methods. The cloning and expression of hsp65 of *Mycobacterium avium* has been described by V. Nagabhushanam, J. Praszkier and C. Cheers, Immunology and Cell Biology (2001) in press. Briefly the sequences of *hsp65* genes from various mycobacterial species were obtained from GenBank, aligned and used to design primers. The open reading frame of the *hsp65* gene of *M. avium* was amplified by PCR. The purified PCR product was inserted into M13tg131B. The insert was re-cloned into M13tg130B, which facilitated sequencing of the complementary strand of the insert. The fragment containing the coding sequence the *hsp65* was moved from the M13tg130B derivative into p2GEX-2T, so that the *hsp65* reading frame was fused in-frame to the 3' end of one of the two copies of the glutathione-S-transferase (GST) of *Schistosoma japonicum*. *E. coli* strain BL21, transformed with the recombinant plasmid p2GEX-hsp65. Expression of the GST fusion was induced by the addition of 0.1mM Isopropylthiogalactoside (IPTG) (Sigma). Bacteria were harvested, washed and lysed. The cell lysate was cleared by centrifugation and the separated cell pellet and supernatant analysed on a 12% SDS-PAGE. The supernatant, which contained the majority of the fusion protein (GST-Hsp65) was filtered and the GST fusion protein purified by binding to glutathione agarose beads according to manufacturers' instructions (Sigma). To cleave Hsp65 from GST, the beads were incubated overnight at room temperature with thrombin (Pharmacia) (10 units per milligram of protein) in 10ml of PBS, then pelleted, the supernatant collected and the protein content estimated spectrophotometrically.

The E7 antigen of Human Papilloma Virus (HPV) was produced by recombinant means. The DNA sequence for the E7 antigen from HPV was cloned into the pGEX-2T vectors. Protein expression was induced by IPTG, and purified using glutathione-sepharose column chromatography. Elution of the bound protein was with a solution of 5mM glutathione in Tris 0.05M, pH 8.0.

The antigen, Cpx, of Porphyromonas gingivalis was prepared according to O'Brien-Simpson et al. [18].

Preparation of oxidised or reduced mannan-antigen conjugates: Mannan (Sigma Chemical Co., St Louis, MO, USA) was coupled to the antigens under oxidising conditions. Mannan at 14mg/ml in 0.1M phosphate buffer pH 6.0 was oxidised with 0.01M sodium periodate for 60 min at 4°C. Ten microlitres of ethandiol (Sigma Chemical Co., St Louis, MO, USA) was added to quench the reaction and the mixture incubated for 30 min at 4°C. This mixture was then passed through a PD-10 column (Pharmacia Biotech, Uppsala, Sweden) equilibrated with bicarbonate buffer pH 9.0. The oxidised mannan, eluted in the 2ml void volume, was mixed with 700µg of the antigen, incubated overnight at room temperature and used without any further purification. Conjugation was confirmed when the conjugates were separated using 12.5% sodium dodecyl sulphate-polyacrylamide gel electrophoresis (SDS-PAGE) and a heterogeneous smear (compared with the single band of uncoupled protein) was observed using Comassie Blue stain. For comparison in some experiments, the oxidised conjugates were reduced with sodium borohydride (NaBH₄) (Aldrich, Castle Hill, NSW, Australia) 1mg/ml overnight at room temperature and used without further purification.

Immunisation with Mannan-antigen conjugates: Mice were lightly anaesthetised with penthrane and 50µl of mannan-antigen conjugate (12µg antigen/mouse in bicarbonate buffer pH9.0, unless otherwise specified), placed onto the nares to be inhaled by the mouse. The same amount of non-conjugated antigen was similarly applied. Unless stated otherwise, this procedure was performed on days 0, 10 and 17 of the experiments. In some experiments mice were given 12µg of mannan-LLO.FP (M-LLO.FP) conjugate intraperitoneally in 0.2ml bicarbonate buffer pH 9.0 on days 0, 10 and 17. Cholera toxin (Sigma Chemical Co., St Louis, MO, USA) was also used for comparative purposes as an adjuvant to act as a positive control in some experiments. One microgram of CT was mixed with 12µg of LLO.FP and administered intranasally (i.n) in a 50µl volume on days 0, 10 and 17.

Collection of samples: Serum samples were collected after mice were bled by cardiac puncture following euthanasia at the end of each experiment. For timecourse experiments mice were placed on a heat box, a small incision made in a lateral vein and 200µl of blood collected with a micropippeter. The serum was subsequently separated by centrifugation. Mouth and vaginal washings were collected from anaesthetised mice, by washing with 50 or 100µl of phosphate buffered saline (PBS) respectively, using a micropippetor. For the collection of faecal samples, mice were placed in cages without sawdust and 2-5 fresh stools were collected per mouse. One ml of PBS/SBTI (PBS containing 0.1mg/ml soybean trypsin inhibitor (Sigma Chemical Co., St Louis, MO, USA)) was added for every 0.1 gm of faeces. The samples were vortexed for 15 minutes and then microcentrifuged at 15,000x g for 15 minutes at 4°C. Phenyl sulphonyl fluoride (PMSF) (Sigma Chemical Co., St Louis, MO, USA) at a final concentration of 1 mM was then added to the supernatants.

Tears were collected by lightly anaethetising the mice with penthrane and a sliver of filter paper was briefly touched on the upper and lower conjunctiva, collecting about 1µl of tears. The paper was then immersed in 100µl PBS-Tween to extract antibody [19]. Lung washings were obtained after mice were euthanased with CO₂. Lungs were washed in situ with 0.5ml PBS through an opening in the trachea. All samples were stored at -20°C prior to assay.

Detection of antibody in the serum and mucosal sites by ELISA: Microtitre plates (Nunc Roskilde, Denmark) were coated overnight at 4°C with 5 µg/ml antigen in carbonate buffer pH 9.1. The wells were then blocked with 2% foetal calf serum (FCS) (Trace Biosciences, Castle Hill, NSW, Australia) in PBS for 1 hour at 37°C. The plates were washed 3 times with 0.08% Tween 20 (BDH Laboratory Supplies, Poole, England) PBS and appropriately diluted samples in 50µl added and incubated for 2 hours at room temperature. After two more washes, antigen-specific IgA was detected by the addition of an anti-mouse IgA affinity purified horse radish peroxidase (HRP) conjugate (Southern Biotechnology Associates Inc. Birmingham, USA) diluted 1/1000 in 0.1% bovine serum albumin (BSA) (CSL, Melbourne, Australia) for 1 hour at room temperature. Antigen specific IgG 1 or IgG2a was detected with the addition of a biotinylated anti-mouse IgG1 or IgG2a conjugate (Caltag Laboratories, Burlinggame, Ca, USA) diluted 1/1000 in 0.1 % BSA. The plates were washed twice more and a streptavidin peroxidase conjugate (Boehringer Mannheim, Mannheim, Germany) added at a 1/1000 dilution in 0.1 % BSA. Following a further two washes, the antibody titres of all the subclasses tested were determined when the substrates containing either 0.4 g/l 3, 3', 5, 5'-tetramethylbenzidine (TMB) (Kirkgaard and Perry Laboratories, Gaithersburg, MD, USA) and 0.02% H₂O₂ or 2,2'-azino-bis(3-ethylbenthiazoline-6-sulphonic acid) (ABTS) (Sigma Chemical Co., St Louis, MO, USA) and 0.03% H₂O₂ were added (50µl/well). Plates were left 10 minutes for colour to develop and the reaction stopped with 2M H₂SO₄ for the TMB substrate or 0.2M citric acid for ABTS. OD at 450 nm (TMB) or 405nm (ABTS) was read in an ELISA reader (Labsystems, Helsinki, Finland). Antibody titres were presented as the highest dilution which yielded an optical density at 450 nm or 405 nm >0.1 OD units higher than normal serum at 1/100 dilution. For calculation of means, the titre was converted to log₁₀ and a geometric mean was derived.

Spleen cell proliferation and cytokine production: Mice were euthanased with CO₂ and spleen cells prepared by passage through an 80 gauge wire mesh sieve. Red blood cells were lysed using Tris-NH₄Cl buffer [20]. After thorough washing, suspensions of spleen cells were cultured in flat-bottomed 96 well plates at a concentration of 2x10⁶/ml in 200µl volumes with and without the MHC class II restricted epitope of LLO₍₂₁₅₋₂₂₆₎ (45µg/ml) for 3 days at 37 °C, 5 % CO₂. For the last 5 hrs the cells were pulsed with 0.25µCi [³H]-TdR (Amersham, Buckinghamshire, U.K) before being harvested onto glass fibre filters (Packard, Meridan, CT, USA) using the Micro 96 harvester (Skatron Instruments, Wokingham, UK) and β emissions counted using a Packard Matrix 9600 (Packard). For IL-2 bioassay, supernatants from similarly-cultured cells were collected after 24 hour culture and IL-2 was assayed by its ability to cause the proliferation of CTLL cells [21].

Elispot: Spleen cells were assayed for the frequency of IFN-γand IL-4 producing cells. Maxisorp 96 well plates (Nunc) were coated overnight at 4 °C with 50µl of 10µg/ml anti-IFN-γ monoclonal antibody HB170 [22] or 100µl of anti-IL-4 monoclonal antibody 11B11 [23] in carbonate buffer pH 9.6. The plates were washed with PBS and blocked with culture medium at 37 °C for 1 hr. Cells (2x10⁵, 1x10⁵, 5x10⁴, 2.5x10⁴ per well) were added to the wells in conjunction with the appropriate antigen (45µg/ml) and incubated for 72 hrs at 37 °C in 5 % CO₂. The cells were removed and cytokines detected with the addition of a biotinylated secondary rat anti-mouse anti-IFN-γ antibody XMG 1.2 [24] or rat anti-mouse anti-IL-4 antibody BVD6 [25] at a 1/1000 dilution. Following the addition of streptavidin alkaline phosphatase (Boehringer Mannheim) the spots were developed using 5-bromo-4-chloro-3-indyl phosphate/nitrobluetetrazolium (BCIP/NBT) (Sigma) tablets. The spots were counted using a dissecting microscope and the frequency determined by averaging the number of spots for triplicate wells.

Statistics: The statistical significance of data was determined by the two-sample ranks test (Wilcoxon-White) or by Student's *t* test based on the log₁₀ titre. Differences with p<0.05 were considered significant.

### Example 2: Effects of Immunisation

A Listeriolysin-mannan (M-LLO) conjugate was prepared as described in Example 1.

LLO specific antibodies in serum M-LLO.FP following intranasal immunisation: To determine whether the intranasal route of immunisation was superior to intraperitoneal in inducing substantial IgA antibody responses, (CBAxBALB/c)F1 mice were immunised i.n or i.p on days 0, 10 and 17 with 12µg M-LLO.FP. Serum was obtained from three mice 7 days after the final immunisation and antibody levels measured by ELISA (Figure 1). Mice immunised i.n with M-LLO.FP, produced antigen-specific IgA to a geometric mean titre of 1/900 (log 2.96 ± 0.21) (Figure 1a), whereas IgA antibody was not detectable after the standard i.p immunisation. It should be noted that i.p immunisation could lead to antibody production, as IgG 1 titres of 1/640 were detected (Figure 1b). However, even with this isotype i.n immunisation was superior, inducing titres in excess of 1/1280.

To further investigate the different antibody classes induced by i.n immunisation, (CBAxBALB/c) F1 mice were immunised i.n with 12µg of M-LLO.FP conjugate on days 0, 10 and 17 and bled on day 24. The serum antibody levels from individual mice were measured by ELISA (Figure 2). IgA serum antibody responses in M-LLO.FP immunised mice (1/5184) were higher than those obtained from mice immunised i.n with 12µg of non-conjugated LLO.FP (1/25) (Figure 2a). A significant difference (p<0.01) in the IgG1 response (Figure 2b), was observed with titres more than 80x higher for M-LLO.FP compared with non-conjugated LLO.FP. A significant difference (p<0.01) was observed between conjugated and non-conjugated LLO.FP with IgG2a titres of 1/32512 and 1/383 respectively (Figure 2c). Infection with *Listeria monocytogenes*, an organism known for its induction of cell mediated immunity, resulted in minimal antibody responses. Overall, the greatest percentage increases were noted for the IgA and IgG2a subclasses of antibody. The experiment was repeated with conjugates prepared on different occasions with three different batches of mannan, and proved to be a very reproducible observation.

Comparison between oxidised mannan and CT as mucosal adjuvants: In previous studies where CT has been used as a mucosal adjuvant in mice, the immunisation regimes adopted have varied [26, 27]. Therefore, to compare the adjuvanticity of mannan and CT the initial experiment was performed using the schedule previously found optimal for mannan [28] of administering antigen and adjuvant on days 0, 10 and 17. (CBAxBALB/c) F1 mice were given either 12µg of M-LLO.FP or CT+LLO.FP i.n. Serum, vaginal washings, mouth washings and faecal samples were collected at the times shown on figure 3 for titration of IgA. IgA titres in negative control groups, including mannan alone, CT alone and normal serum, were not more than the limit of detection and are not shown.

Oxidised mannan was a consistently better mucosal adjuvant than CT when administered with LLO.FP. Immunisation with M-LLO.FP induced a peak geometric mean IgA titre of 1/667 in serum with some titres in excess of 1/1280. This compared with 1/165 for CT+LLO.FP (Figure 3a ). LLO.FP alone induced a mean titre of only 1/69.

The superior efficacy of M-LLO.FP was also observed at distant mucosal sites. IgA titres in vaginal washings (Figure 3b) were more variable than in serum, but individual mice reached titres > 1 /320 (geometric mean = 1/195) after M-LLO.FP, compared with a mean of 1/55 for CT + LLO.FP. LLO.FP alone induced a mean titre of 1/37. IgA titres in the saliva (Figure 3c) of M-LLO.FP immunised mice were only detectable on day 41 of the experiment with a mean of 1/10 and a high of 1/28. Titres, although low, were higher than those observed for LLO.FP or CT+LLO.FP. In both instances, CT was quite a poor adjuvant.

In a second series of experiments (Figure 4), the immunisation schedule was extended to 0, 28 and 56 days, with doses as before, to test whether a longer time between prime and boost would favour either adjuvant. Again the response to M-LLO.FP produced higher titres than CT + LLO.FP. The peak geometric mean titres following M-LLO.FP were 1/1040 in serum and 1/256 in vaginal washings. This compared with 1/44 and 1/22 respectively following CT+LLO.FP. Although differences were often not statistically significant because of the variability of individual mice, oxidised mannan was consistently the better adjuvant under two different immunisation regimes.

IgA titres were examined over a considerable timecourse in these experiments. In general, they did not reach substantial levels until after three injections, whether of M-LLO.FP or CT + LLO.FP. IgA persisted at peak titre in the serum for more than three weeks after the last immunisation, particularly of M-LLO.FP (Figure 3a, 4a). IgA production in the vagina (Figure 3b, 4b) also required three immunisations and these titres were more variable than in serum, and generally less sustained.

To extend the investigation of mucosal IgA to other sites, (CBAxBALB/c) F 1 mice were immunised i.n. with M-LLO.FP as before and tears and lung washings collected on days 24 and 35 respectively. Mice immunised with M-LLO.FP conjugate produced significantly higher titres of IgA (p<0.01) in tears when compared with the unconjugated controls (geometric means titres: M-LLO.FP 1/121 compared with 1/25 for LLO.FP alone) (Figure 5a). The same trend was observed in the lungs at day 35 (Figure 5b). Significantly higher titres of IgA were detected in mice immunised with M-LLO.FP (1/975) compared with LLO.FP alone (1/38) (p<0.05).

### Example 3: Influence of Conjugation of Mannan to Antigen on IgA Induction

To investigate whether there was a need for the mannan to be conjugated to LLO.FP or whether the adjuvant effect could be achieved when mannan was simply mixed with antigen, mice were immunised with the M-LLO.FP conjugate or with a mannan+LLO.FP mixture (mannan and 12 µg of LLO.FP mixed together). (CBAxBALB/c)F1 mice were immunised i.n on days 0, 10 and 17. Serum and vaginal washings were taken from individual mice on day 24 of the experiment. IgA titres were subsequently determined by ELISA (Table 1). The results showed that a conjugate provides a better adjuvant effect. The geometric mean antibody titre in the serum for conjugated LLO.FP was >1/1000. This was significantly higher than the mannan+LLO.FP mixture which produced an average titre of 1/34. Notwithstanding this some antibody is obtained from the mannan antigen admixture. A similar pattern was seen in the antibody response at a distant mucosal site, namely the vagina, with titres of 1/195 for the conjugate and 1/45 for the mixture. All negative controls produced undetectable levels of IgA with small titres of antibody observed in vaginal washings for the LLO.FP alone group (1/14).

Earlier experiments with mannan-conjugated proteins injected i.p showed that conjugates produced under reduced conditions were better at inducing antibody than were oxidised conjugates [13]. Therefore, (CBAxBALB/c) F1 mice were immunised i.n with 12 µg of M-LLO.FP conjugate (oxidised form) or 12µg of M-LLO.FP conjugate (reduced form). The results indicated that the oxidised form of the M-LLO.FP conjugate given i.n induced higher titres of IgA, IgG1 and IgG2a in the serum (Figure 6a, b and c) compared with the reduced form. All four mice immunised with M-LLO.FP (oxidised form) had higher titres than the corresponding four mice given the reduced form of M-LLO.FP, with the exception of one high responder mouse in the group given reduced mannan. Significant differences (p<0.02) applied to all the subclasses assayed (geometric mean titres: IgA = 1/1380 for oxidised, 1/275 for reduced; IgG1 = 1/3388 for oxidised, 1/813 for reduced; IgG2a = 1/1660 for oxidised, 1/173 for reduced).

### Example 4: Mannan as Adjuvant for other Antigens

To demonstrate that the use of mannan as a mucosal adjuvant was applicable to other antigens mannan was conjugated to the 19 kDa lipoprotein of Mycobacterium tuberculosis and to *Mycobacterium avium* antigen *hsp65* as set forth in Example 1. C57B1/10 mice were immunised i.n with 12 µg of M-19FP conjugate on days 0, 10 and 17. On day 24 of the experiment the mice were euthanased and bled by cardiac puncture. Mice were similarly immunised with mannan hsp65. Serum was separated and IgA titres determined by ELISA (Figure 7a). The four mice immunised with M-19FP produced significantly higher titres of IgA (geometric mean 1/1530) when compared with mice given 12µg of 19 kDaFP (titres of IgA <1/100). The results for mice immunised with mannan-hsp65 are shown in Figure 7(b). IgA titres for the immunised mice were well above that of the normal mice.

The examples above present mannan as a novel mucosal adjuvant which, when administered intranasally, induced high mucosal and serum titres of IgA, IgG 1 and IgG2a specific for recombinant protein antigens with which it was administered. Oxidised mannan was previously used conjugated to the breast cancer antigen MUC1 and injected i.p into mice where it induced CTL and a Th1 cytokines, protecting against tumours expressing MUC1 [12, 13]. In that context it induced a poor antibody response, dominated by IgG2a. It was the change to intranasal administration in the current experiments which resulted production of IgA and other classes of antibody in serum and mucosal secretions.

The induction of IgA responses on mucosal surfaces is a highly desirable outcome in immunisation against a wide spectrum of diseases. Amongst other examples, mucosal antibodies and particularly IgA, have been shown to protect against influenza in the lung [29], against *Helicobacter pylori* in the stomach [30], against *Haemophilis influenzae* in the ear [31] and against *Chlamidia trachomatis* in the genital tract [32]. Induction of IgA and other mucosal antibodies following intramuscular or subcutaneous immunisation with conventional vaccines is poor [33, 33a]. Therefore oxidised mannan may be a valuable adjuvant if coupled to protective antigens from a wide variety of infectious agents.

Cholera toxin has become the most extensively studied mucosal adjuvant in experimental models, although its relative toxicity has so far precluded it from approval as a human adjuvant [34]. In the present experiments, immunisation with mannan-conjugated LLO intranasally induced superior IgA and IgG2a responses in the mucosa and serum compared with CT plus LLO or LLO alone. The difference in the IgG1 response was less marked, but still higher for the mannan adjuvant. Titres of antibody following immunisation with mannan conjugates were not only higher than with CT, they were more sustained. Mannan has the further advantage over CT in being non-toxic and already approved for human use and is currently involved in human trails for cancer therapy [14]. As with the present observations on mannan, a mixture of the B subunit of CT (CTB) with antigen resulted in higher titres of IgA in the serum and mucosa when given to mice intranasally rather than intraperitoneally [33]. It is widely believed that this influence of the route of exposure is a function of the mucosal antigen presenting cells.

Two interesting observations were made when analysing the form of the antigen/adjuvant in the present experiments. The first showed that oxidised mannan conjugated to the antigen (LLO.FP or 19kDaFP) facilitates the adjuvant effect. This confirms the observations with the MUC 1 studies where conjugation of oxidised mannan to the MUC 1 antigen induced better immune responses [13]. The second observation contrasts with the MUC 1 studies because the oxidised form of the M-LLO.FP induced maximum antibody production. Immunisation with the oxidised form of mannan-MUC 1 resulted in low antibody levels [13].

How, then, does mannan act as an adjuvant? It has been shown that antigens bearing mannose residues are bound to the mannose receptors on antigen presenting cells (APC), facilitating uptake into the MHC Class II pathway for efficient antigen presentation [35, 36, 37]. Oxidised mannan-MUC1 induced both Class I restricted CTL and a Th1 response [12]. It has been suggested that oxidised mannan-MUC1, by virtue of the aldehyde residues created by the oxidation process, escapes the phagocytic pathway and is transported into the MHC Class I pathway inducing CTL [15]. This pathway would not be open to reduced complexes, where aldehyde residues are reduced to alcohols by the action of boral hydrate. The exact mechanism by which aldehyde acts is unknown. Curiously, the inventors did not detect the induction of Class I-restricted CTL in experiments with M-LLO.FP injected i.p. Furthermore, the response to the inventor's antigens given intranasally was not classically either Th1 or Th2, since IgG1 and IgA (generally considered Th2 responses) and IgG2a (Th1) were all elevated. This was confirmed by cytokine assays where both IFN-γ and IL-4 were elevated. This aspect is further explored in Example 5.

The mechanism by which M-LLO.FP and M-19FP induce such excellent antibody responses after i.n immunisation is unknown. Others have shown preferential induction of Th2 responses following intranasal instillation of leishmania antigens, even in strains of mouse which are genetically constrained to produce a typical Th1 response to these organisms injected parenterally [38]. Without wishing to be bound by theory it may be that alveolar macrophages or dendritic cells, which have been shown to present antigens efficiently in other systems [39], are playing a significant role in the induction of these IgA responses.

Induction of mucosal immunity begins with the uptake of antigen by membranous (M) cells (specialised epithelial cells) on the mucosal surface. These cells either process and present antigen to underlying T-Cells or B-cells themselves or transport antigen to parenchymal macrophages, dendritic cells and B-cells. Once interaction of the antigen presenting cell (APC) with T and B lymphocytes has occurred, an immune response or mucosal tolerance may result. Immune responses generally involve antibody production, with IgA the predominant antibody isotype. Antigen sensitised immune cells are then circulated to other systemic and mucosal sites for expansion of effector mechanisms [40]. The fact that there is a preferential circulation of T cells activated at mucosal sites to return to the same or other mucosal sites accounts for the induction of IgA at remote mucosal surfaces.

### Example 5: Oxidised mannan-listeriolysin O conjugates induce Thl/Th2 cytokine responses after intranasal immunisation

Clearance of infectious organisms does not always require polarised Th1 or Th2 responses. It may in fact be advantageous for both a Th 1 and Th2 response to be elicited for effective protection against an invading pathogen. It was the aim of this Example to investigate oxidised mannan as a possible Th1/Th2 adjuvant.

### 5.1 Materials and Methods

Mice, production of antigens, conjugates and immunisation were the same as Example 1. Spleen cell proliferation, cytokine production and Elispot assays were as described in Example 1.

### 5.2 Results

### a. Comparison of intranasal and intraperitoneal immunisation

Having established that the intranasal route of administration produced better antibody responses than those measured after intraperitoneal immunisation as described in Example 2, a direct comparison between the two routes of immunisation was undertaken to determine if a similar pattern could be established for cellular responses. (CBAxBALB/c) F1 mice were immunised i.n. or i.p. on days 0, 10 and 17 with 12 µg of M-LLO.FP. On day 24 the mice were sacrificed and spleens removed. Spleen cell preparations were used to establish proliferation, IL-2 and IFN-γ ELISPOT assays. Spleen cells were cultured in the presence of the MHC class II restricted epitope of LLO₍₂₁₅₋₂₂₆₎ and incorporation of [³H]-TdR measured to determine the proliferative response (Figure 8a). Mice immunised with M-LLO.FP intranasally produced counts significantly higher than the group of mice which had the M-LLO.FP administered by the intraperitoneal route (3972±502 vs 983±145, p<0.002). The latter approximated background levels. LLO.FP without adjuvant by either route induced background levels of proliferation only.

The ability of spleen cells to produce IL-2 is shown in Figure 8b. Spleen cells were cultured in the presence of LLO₍₂₁₅₋₂₂₆₎ or LLO.FP (45µg/ml) and supernatants harvested at 24 hours. The ability of the supernatants to maintain the IL-2 dependent CTL cell line was determined by the incorporation of [³H]-TdR. Mice immunised with M-LLO.FP intranasally produced counts of 1219±268 (LLO₍₂₁₅₋₂₂₆₎) and 4523±689 (LLO.FP). These values were significantly higher (p<0.01, p<0.002) than the group of mice which received the M-LLO.FP administered by the intraperitoneal route with geometric means of 182±73 (LLO₍₂₁₅₋₂₂₆₎) and 417±40 (LLO.FP), which approximated background levels. LLO.FP without adjuvant by either route induced background levels of IL-2.

Importantly this pattern was also observed with the IFN-γ ELISPOT (Figure 8c). Spleen cells were cultured in the presence of LLO.FP and the frequency of IFN-γ-producing cells determined by ELISPOT. Mice immunised with M-LLO.FP intranasally produced a high frequency of IFN-γ-producing cells/10⁶ spleen cells (58±20), significantly higher (p<0.05) than the group of mice which had the M-LLO.FP administered by the intraperitoneal route (10±8) which approximated background levels. Mice given LLO.FP by either route without adjuvant recalled levels of IFN-γ producing cells just above background.

### b. Dose response

Intranasal immunisation with 12µg of M-LLO.FP resulted in significant proliferative and Th1 cytokine (IL-2 and IFN-γ) responses, but Th1 responses are often favoured by lower doses of antigen [41]. Therefore, (CBAxBALB/c) F1 mice were immunised i.n. on days 0, 10 and 17 with 12µg, 6µg or 3µg of M-LLO.FP and on day 24 the mice were sacrificed and spleens removed. Proliferative responses and the frequency of IFN-γ-producing cells from spleen cell preparations were measured. Proliferative responses after in vitro stimulus with the MHC class II restricted epitope of LLO indicated that intranasal immunisation with the 12µg dose was significantly higher than the 6µg (p<0.02) and 3µg (p<0.01) dose of M-LLO.FP (6385±1506, 1962±649 and 1630±375 respectively). Cells from unconjugated doses of LLO.FP produced levels of proliferation slightly above background (results not shown).

A similar pattern of results was observed for the production of IFN-γ. The IFN-γ producing cells /10⁶ spleen cells for the 12µg dose was significantly higher than for the 6µg (p<0.01) and 3µg (p<0.01) dose of M-LLO.FP (97±20, 32±8 and 12±6 respectively). Mice given the unconjugated control antigen all produced fewer than 50 IFN-γ producing cells/10⁶ spleen cells (results not shown).

### c. Comparison of the oxidised and reduced form of M-LLO.FP

Earlier studies with mannan-conjugated proteins injected intraperitoneally showed that conjugates produced under oxidising conditions were better at inducing Th1 responses compared to conjugates produced under reduced conditions [13] that favoured Th2 responses. To test this hypothesis (CBAxBALB/c) F1 mice were immunised i.n. with 12µg of M-LLO.FP conjugate (oxidised form) or 12µg of M-LLO.FP conjugate (reduced form) on days 0, 10 and 17 and mice sacrificed on day 24. A comparison of the two forms of conjugate for their ability to produce the Th1 cytokine IFN-γ or the Th2 cytokine IL-4 was performed using the ELISPOT assay. Spleen cells were cultured in the presence of LLO.FP and the frequency of IFN-γ or IL-4 producing spots determined (Figure 9). The results clearly indicated that the oxidised form of the conjugate produced significantly higher frequencies of both IFN-γ and IL-4 producing cells (107±12 and 123±12 respectively) when compared to the reduced form (32±10 and 57±38) (p<0.02, p<0.05). There was no indication that, using this antigen and route of immunisation, that the oxidised form induced Th1 and the reduced form induced Th2 cytokines. However this may be dose-dependent.

### 5.3. Discussion

These experiments present oxidised mannan as an adjuvant which, when administered intranasally, induced proliferative responses, IL-2 production, IFN-γ production and IL-4 production by cultured lymphocytes. Comparison of intranasal and intraperitoneal routes of immunisation revealed the superior efficacy for induction of these immune responses by the intranasal route. As most proteins administered to the mucosal surface without adjuvant fail to induce systemic or mucosal immune responses, oxidised mannan may be used as an adjuvant to induce both Th1 and Th2 immune responses. It offers the great advantage as an adjuvant of being non-toxic and already approved for human use in the context of breast-cancer therapy.

There is evidence in the literature that suggests that variations in antigen dose can polarise immune responses, inducing either DTH or antibody responses depending on antigen concentration. It is generally believed that low doses of antigen favour Th1 responses and high doses of antigen favour Th2 responses [41]. The results described in this study do not support this. Low doses of M-LLO.FP conjugate i.n. were unable to induce Th1 responses. Interestingly, the highest dose of M-LLO.FP conjugate was responsible for increases in the Th1 cytokine IFN-γ. These results contradict earlier observations where immunisation with low doses of mannan-conjugates favoured Th1 responses [28].

It appears that oxidised mannan administered intranasally can be an effective adjuvant for the induction of Th1 and Th2 responses as seen with the two model antigens used in this Example. This ability is of particular interest with infections such as C. *trachomatis* or *H. pylori* where both cell mediated and humoral responses appear necessary for control of infection. In the case of *C. trachomatis,* cell mediated responses alone appear to contribute to immunopathology whereas antibody responses are not sufficient to control infection [42, 43]. It has been suggested in the case of *H. pylori* that Th1 and Th2 responses are required at different stages for control of infection. The early response is dominated by Th 1 and the late by Th2 [44]. Candidate vaccines such as those contemplated by the present invention with the ability to produce both Th1 and Th2 responses might therefore prove to be effective against both pathogens.

### Example 6: Immunisation with Rotavirus VP5 Antigen Conjugated Mannan

Mice and immunisation thereof were as described in Example 1. Samples of serum, tears and/or stools were collected as described in Example 1 at days 24 and 34 after immunisation. On day 35 the mice were sacrificed and lung washings were collected as shown in Example 1.

Detection of antibody in the samples was carried out as described in Example 1. The results are represented in Figures 11 and 12. Figure 11 shows that in mice immunised intranasally with 6ug VP5₂₄₆₋₂₇₄ -mannan, titres of serum IgA, IgG1 and IgG2a were significantly higher than in mice immunised intranasally with the antigen alone, at 24 and 35 days after immunisation.

Figure 12 shows the presence of IgA in tears collected 24 days after immunisation with the antigen-mannan conjugate, and in the stools collected at day 34. No antibody was detected in mice receiving antigen alone. IgA in lung washings was also significantly higher than in samples from animals injected with the antigen alone.

### Example 7: Protection against Porphyromonas gingivalis lesions:

The antigen used was the major virulence factor of Porphyromonas gingivalis, the extracellular complex of Arg- and Lys-specific proteases and adhesions designated the RpgA-Kgp complex (formerly the PrtR-PrtK complex and abbreviated Cpx), prepared and conjugated with mannan as described in Example 1. Cpx antigen (12µg) was administered intranasally on days 0, 10 and 17. Alternatively, the Cpx antigen was administered subcutaneously in incomplete Freunds adjuvant as previously described [18]. Mice were infected with 8x10⁹ cells of Porphyromonas gingivalis subcutaneously in the abdomen on day 30 and lesion sizes measured over 14 days. Lesion sizes were statistically analysed using the Kruskal-Wallis test and the Mann-Whitney U-Wilcoxon rank sum test with a Bonferroni correction.

Groups of 5 C57B1/10 mice were immunised with Cpx-mannan given intranasally or Cpx in incomplete Freund's adjuvant (IFA) subcutaneously. Control mice received the respective adjuvants alone. Results of protection against challenge with Porphyromonas gingivalis are shown in Figure 13. Cpx linked to mannan afforded complete protection. IFA, the standard adjuvant used in studies of Porphyromonas gingivalis vaccination afforded only partial protection. Interestingly, the serum antibody titres, although raised in the mice immunised with Cpx-mannan, was higher in the mice immunised with Cpx in IFA. Both had significant adjuvant effects (p<0.05). This emphasises the point that, particularly where inflammatory lesions are critical to disease, antibody titre may not be an accurate predictor of protection.

### Example 8: Immunisation with HPV-E7 :

Mice were immunised with HPV-E7 conjugated to oxidised mannan, E7 conjugated to reduced mannan and with E7 alone, as per Example 1. The results (Figure 15) show no substantial difference between the oxidised and reduced forms although this may have been due to dose levels.

### Example 9: Immunisation with other Antigens

Antigens used in accordance with the invention may also be produced by conventional peptide synthesis after selecting putative immunogenic peptides. The putative immunogenic peptides may be selected from published studies or may be peptides suspected of being immunogenic based on a skilled person's knowledge of the art. The putative immunogenic peptides are then coupled to a carrier protein which is, in turn, conjugated to mannan.

Alternatively, plasmids containing genes encoding selected antigens are expressed in E. coli. The proteins coupled to mannan are administered and the antibody response at the site most relevant to protection is examined. Examples of proteins that can be tested are as follows:

| ORGANISM | DISEASE | PROTECTIVE ANTIGEN | SITE OF ANTIBODY |
|---|---|---|---|
| Influenza virus | Flu | Haemagglutinin | Lungs |
| Helicobacter pylori | Stomach ulcers, cancer | Recombinant proteins, urease | Stomach |
| Rotavirus | Gastroenteritis in infants | Recombinant VP4 protein and its fragment VP5 and other outer capsid proteins | Intestine |
| Chlamydia trachomatis | Sexually transmitted disease, pelvic inflammatory disease, non-specific urethritis, trachoma | Major outer membrane protein | Vagina, Eye |

Mannan-antigen complexes are encased in various timed-delivery capsules and delivered intranasally, orally or rectally. At intervals, serum, saliva, lung or vaginal washings will be collected, and ELISA are used to measure IgG1, IgG2a, IgE and IgA.

### Conclusion

In this study the inventors have shown the application of the invention to a number of antigens. In addition, they have shown that antibodies to the antigens have been detected at a number of sites including serum, lungs, vagina, lachrymal glands and colon. These results are surprising for a number of reasons. Past studies have demonstrated that mice immunised by standard methods with oxidised mannan-human MUC1 conjugate produced cellular immunity but little humoral immunity (i.e. antibody). Whereas when the reduced form of the same conjugate was administered to mice by standard methods only a serum antibody response was found. Other studies have shown that when various antigens (MUC1, lysteriolysin) conjugated to oxidised mannan were administered to humans, mice and monkeys mostly IgG1 antibodies were found. There was no IgA response. Given this background it was extremely surprising that intranasal administration of mannan-antigen gave rise to secreted IgA.

The work done by the inventors indicates that intranasal administration of mannan and antigen may advance vaccine development for sexually transmitted diseases such as chlamydia or human immunodeficiency virus, gum disease, eye diseases, other mucosally acquired infections like influenza and rotavirus or even have veterinary application with uses in animal infections or pest control. The fact is that this is a novel, non-toxic adjuvant that induces systemic and mucosal antibody responses superior to the standard mucosal adjuvant CT.

### REFERENCES

[1] Williams R. C., and R. J. Gibbons. 1972. Inhibition of bacterial adherence by secretory immunoglobulin A: a mechanism of antigen disposal. Science 177: 697-699.
[2] Hajishengallis G., E. Nikolova, and M. W. Russell. 1992. Inhibition of Streptococcus mutans adherence to saliva-coated hydroxyapatite by human secretory immunoglobulin A (S-IgA) antibodies to cell surface protein antigen I/II: reversal by IgAl protease cleavage. Infect Immun 60: 5057-5064.
[3] Gilbert J. V., A. G. Plaut, B. Longmaid, and M. E. Lamm. 1983. Inhibition of microbial IgA proteases by human secretory IgA and serum. Mol Immunol 20: 1039-1049.
[4] Outlaw M. C., and N. J. Dimmock. 1990. Mechanisms of neutralization of influenza virus on mouse tracheal epithelial cells by mouse monoclonal polymeric IgA and polyclonal IgM directed against the viral haemagglutinin. J Gen Virol 71: 69-76.
[5] Elson C. O., and Ealding W. 1984. Generalized systemic and mucosal immunity in mice after mucosal stimulation with cholera toxin. J. Immunol 132: 2736-2741.
[6] de Hann L., Verweij W., Agsteribbe E., and Wilschut J. 1998. The role of ADP-ribosylation and G(M)1-binding activity in the mucosal immunogenicity and adjuvanticity of Escherichia coli heat-labile enterotoxin and Vibrio cholerae cholera toxin. Immunol Cell Biol 76: 270-279.
[7] Wiedermann U, John-Schmid B, Lindblad M et al, 1999, Int Immunol. 11(10): 1717-1724
[8] Cardenas-Freytag L, Cheng E, Mayeux P et al, 1999, Infect Immun 67 (2): 826-833
[9] Hamajima K, Sasaki S, Fukushima J, et al 1998, Clin Immunol Immunopathol 88 (2) : 205-210
[10] Sasaki S, Hamajima K, Fukushima J, et al 1998, Infect Immunol 66 (2): 823-826
[11] Robert-Guroff M, 1990, Int Rev Immunol 7: 15-30]
[12] Apostolopoulos V., B. E. Loveland, G. A. Pietersz, and I. F. McKenzie. 1995. CTL in mice immunized with human mucin 1 are MHC-restricted. J Immunol 155: 5089-5094.
[13] Apostolopoulos V., G. A. Pietersz, B. E. Loveland, M. S. Sandrin, and I. F. McKenzie. 1995. Oxidative/reductive conjugation of mannan to antigen selects for T1 or T2 immune responses. Proc Natl Acad Sci U S A 92: 10128-10132.
[14] Karanikas V., L. A. Hwang, J. Pearson, C. S. Ong, V. Apostolopoulos, H. Vaughan, P. X. Xing, G. Jamieson, G. Pietersz, B. Tait, R. Broadbent, G. Thynne, and I. F. McKenzie. 1997. Antibody and T cell responses of patients with adenocarcinoma immunized with mannan-MUC1 fusion protein. J Clin Invest 100: 2783-2792.
[15] Apostolopoulos V P. G. A., Siamon G, Martinez-Pomares L, McKenzie IFC. 2000. Alhehyde-mannan antigen complexes target the MHC Class I antigen presentation pathways. Eur J Immunol 30, 1714-1723.
[16] Smith D. B., and K. S. Johnson. 1988. Single-step purification of polypeptides expressed in Escherichia coli as fusions with glutathione S-transferase. Gene 67:31-40.
[17] Denisova E., W. Dowling, R. LaMonica, R. Shaw, S. Scarlata, F. Ruggeri and E.R. Mackow, 1999. Rotavirus capsid protein VP5 permeabilizes membranes. J. Virol. 73: 3147-3153.
[18] O'Brien-Simpson N. M., Paolini R. A., and Renolds E. C. 2000. RgpA-Kgp peptide-based immunogens provide protection against Porphyromonas gingivalis challenge in a murine lesion model. Inf Immun 68: 4055-4063.
[19] Preston M.J., K.A. Kemacki, J.M. Berk, L.D. Hazlett and R.S. Berk, 1992. Kinetics of serum, tear and corneal antibody responses in resistant and susceptible mice intracomeally infected with Pseudomonas aeruginosa. Infect. Immun. 60: 885-891.
[20] Boyle, W. An extension of the 51 Cr-release assay for the estimation of mouse cytotoxins. Transplantation 1968, 6, 761-764.
[21] Gillis, S., Ferm, M.M., Ou, W. and Smith, K.A. T cell growth factor: parameters of production and a quantitative microassay for activity. J. Immunol. 1978, 120, 2027-2032.
[22] Havell, E.A. Augmented induction of interferons during Listeria monocytogenes infection. J. Infect. Dis. 1986, 153, 960-969.
[23] Cherwinski, H.M., Schumacher, J.H., Brown, K.D. and Mosmann, T.R. Two types of mouse helper T cell clone. III. Further differences in lymphokine synthesis between Th1 and Th2 clones revealed by RNA hybridization, functionally monospecific bioassays, and monoclonal antibodies. J. Exp. Med. 1987, 166, 1229-1244.
[24] Taguchi, T., McGhee, J.R., Coffman, R.L., Beagley, K.W., Eldridge, J.H., Takatsu, K. and Kiyono, H. Detection of individual mouse splenic T cells producing IFN-gamma and IL-5 using the enzyme-linked immunospot (ELISPOT) assay. J. Immunol. Methods 1990, 128, 65-73.
[25] Ohara, J. and Paul, W. E. Production of a monoclonal antibody to and molecular characterization of B-cell stimulatory factor-1. Nature 1985, 315, 333-336.
[26] Hvalbye B. K., I. S. Aaberge, M. Lovik, and B. Haneberg. 1999. Intranasal immunization with heat-inactivated Streptococcus pneumoniae protects mice against systemic pneumococcal infection. Infect Immun 67:4320-4325.
[27] Vadolas J., J. K. Davies, P. J. Wright, and R. A. Strugnell. 1995. Intranasal immunization with liposomes induces strong mucosal immune responses in mice. Eur J Immunol 25: 969-975.
[28] Pietersz, G.A., Li, W., Popovski, V., Caruana, J.A., Apostolopoulos, V. and McKenzie, I.F., 1998. Parameters for using mannan-MUC1 fusion protein to induce cellular immunity. Cancer Immunol. Immunother. 45:321-326.
[29] Tamura S., H. Funato, Y. Hirabayashi, Y. Suzuki, T. Nagamine, C. Aizawa, and T. Kurata. 1991. Cross-protection against influenza A virus infection by passively transferred respiratory tract IgA antibodies to different hemagglutinin molecules. Eur J Immunol 21: 1337-1344.
[30] Blanchard TG N. J. a. C. S. Effects of microbes on the immune system: Immunity against Helicobacter pylori; Lippincott Williams and Wilkins: Philadelphia, 2000, pp 623-629.
[31] Kodama S., S. Suenaga, T. Hirano, M. Suzuki, and G. Mogi. 2000. Induction of specific immunoglobulin A and Th2 immune responses to P6 outer membrane protein of nontypeable Haemophilus influenzae in middle ear mucosa by Intranasal immunization. Infect Immun 68: 2294-2300.
[32] Su H., K. Feilzer, H. D. Caldwell, and R. P. Morrison. 1997. Chlamydia trachomatis genital tract infection of antibody-deficient gene knockout mice. Infect Immun 65: 1993-1999.
[33] Hirabayashi Y., H. Kurata, H. Funato, T. Nagamine, C. Aizawa, S. Tamura, K. Shimada, and T. Kurata. 1990. Comparison of intranasal inoculation of influenza HA vaccine combined with cholera toxin B subunit with oral or parenteral vaccination. Vaccine 8: 243-248.
[33a] Muszkat M., A.B. Yehuda, M.H. Schein, Y. Friedlander, P. Naveh, E. Greenbaum, M. Schlesinger, R. Levy, Z. Zakay-Rones, and G. Friedman. 2000. Local and systemic immune response in community-dwelling elderly after intranasal or intramuscular immunization with inactivated influenza vaccine [In Process Citation]. J Med Virol 61:100-106.
[34] Levine M. M. 1987. Escherichia coli that cause diarrhea: enterotoxigenic, enteropathogenic, enteroinvasive, enterohemorrhagic, and enteroadherent. J Infect Dis 155:377-389.
[35] Sallusto F., M. Cella, C. Danieli, and A. Lanzavecchia. 1995. Dendritic cells use macropinocytosis and the mannose receptor to concentrate macromolecules in the major histocompatibility complex class II compartment: downregulation by cytokines and bacterial products. J Exp Med 182: 389-400.
[36] Tan M. C., A. M. Mommaas, J. W. Drijfhout, R. Jordens, J. J. Onderwater, D. Verwoerd, A. A. Mulder, A. N. van der Heiden, D. Scheidegger, L. C. Oomen, T. H. Ottenhoff, A. Tulp, J. J. Neefjes, and F. Koning. 1997. Mannose receptor-mediated uptake of antigens strongly enhances HLA class II-restricted antigen presentation by cultured dendritic cells. Eur J Immunol 27: 2426-2435.
[37] Engering A. J., M. Cella, D. Fluitsma, M. Brockhaus, E. C. Hoefsmit, A. Lanzavecchia, and J. Pieters. 1997. The mannose receptor functions as a high capacity and broad specificity antigen receptor in human dendritic cells. Eur J Immunol 27: 2417-2425.
[38] Constant S. L., K. S. Lee, and K. Bottomly. 2000. Site of antigen delivery can influence T cell priming: pulmonary environment promotes preferential Th2-type differentiation. Eur J Immunol 30: 840-847.
[39] Hamilton-Easton A., and M. Eichelberger. 1995. Virus-specific antigen presentation by different subsets of cells from lung and mediastinal lymph node tissues of influenza virus-infected mice. J Virol 69: 6359-6366.
[40] Mestecky J. 1987. The common mucosal immune system and current strategies for induction of immune responses in external secretions. J Clin Immunol 7:265-276.
[41] Lagrange, P.H., Mackaness G.B. and Miller, T.E. Influence of dose and route of antigen injection on the immunological induction of T cells. J. Exp. Med. 1974, 139, 528- 542.
[42] Beagley, K. W. and Timms, P. Chlamydia trochomatis infection: incidence, health costs and prospects for vaccine development. J. Reprod Immunol. 2000, 48, 47-68.
[43] Stagg, A. J. Vaccines against chlamydia: approaches and progress. Mol Med Today. 1998, 4, 166-173.
[44] Mohammadi, M., Czinn, S., Redline, R. and Nedrud, J. Helicobacter-specific cell-mediated immune responses display a predominant Th1 phenotype and promote a delayed-type hypersensitivity response in the stomachs of mice. J. Immunol. 1996, 156, 4729-4738.

**Table 1: Requirement for conjugation of mannan and LLOFP for IgA production:**

| Antigen/Adjuvant^{a} | Log₁₀ IgA antibody titre | |
|---|---|---|
| | Serum^{b} | Vaginal washings^{b} |
| M-LLO.FP (linked) | 3.00 ± 0.19^{c} | 2.29 ± 0.70^{c} |
| M + LLO.FP (mixed) | 1.53 ± 0.49 | 1.65 ± 0.48 |
| LLO.FP | 1.15 ± 0.30 | 1.15 ± 0.11 |
| Mannan | <1 | 1.12 ± 0.15 |
| Normal | <1 | 1.08 ± 0.10 |

| | | |
|---|---|---|
| ^{a} (CBAxBALB/c) mice were immunised intranasally with M-LLOFP, LLOFP, mannan mixed with LLOFP (unconjugated) or mannan alone on day 0, 10 and 17. Serum samples and vaginal washings obtained on day 24 from 4 individual mice. ^{b} Individual IgA titres were determined by ELISA. Antibody titres were converted to log₁₀ and geometric means and standard deviations were derived. ^{c} Significantly different from mannan+LLOFP (mixed), p<0.05 | | |

## Claims

1. Use of a composition comprising an antigen and a carbohydrate polymer adjuvant comprising mannose, wherein said polymer
a) comprises mannose monomers oxidized to produce aldehyde groups, and
b) is covalently bonded to the antigen;
for the preparation of a vaccine for intranasal administration to generate an immune response to the antigen comprising a secretory immune response.

2. The use of claim 1, wherein the immune response comprises a humoral immune response.

3. The use of claim 1 or claim 2, wherein the immune response comprises an IgA response.

4. The use according to any one of claims 1 to 3, wherein immune response comprises increased antibody levels at mucosal sites.

5. The use according to any one of claims 1 to 4, wherein immune response comprises increased antibody levels in serum.

6. The use according to any one of claims 1 to 5, wherein immune response comprises stimulation of mediators of Th1 and/or Th2 cellular immunity.

7. The use according to any one of claims 1 to 6, wherein IgE production is reduced or unchanged.

8. The use according to any one of claims 1 to 7, wherein the antigen is selected from the group consisting of the following or immunogenic portions thereof: pollens, allergens, bacteria, viruses, yeast, fungi, protozoa or other microorganisms including pathogens of humans, animals and plants.

9. The use of claim 8, wherein the antigen is selected from the group consisting of influenza virus, haemagglutinin of influenza, *Porphyromona gingivalis*, proteinase and adhesin epitopes of *Porphyromona gingivalis*, *Helicobacter pylori,* urease of *Helicobacter pylori,* rotavirus, recombinant VP5 protein of rotavirus, HIV, gp120 of HIV, RSV, surface proteins of RSV and ovalbumin peptides and *Listeria monocytogenes, Mycobacterium tuberculosis, Mycobacterium avium,* BCG, influenza nucleoprotein, Respiratory Syncyticial virus (RSV) F or G proteins, *Candida albicans* and *Chlamydia trachomatis* or outer membrane proteins thereof, *Neisseria meningitidis* class 1 outer protein, Herpes simplex virus type I glycoprotein G or gp D or CP27, Human Papilloma Virus, Murray valley encephalitis virus E glycoprotein, antigenic portions thereof and derivatives thereof.

10. The use of claim 8 or claim 9, wherein the antigen is selected from the group consisting of HIV antigens, malaria antigens, antigens from microorganisms which cause venereal disease, common cold or influenza and antigens which induce asthma.

## Patentansprüche

1. Verwendung einer Zusammensetzung, umfassend ein Antigen und ein Kohlenhydrat-Polymer-Adjuvans, umfassend Mannose, wobei das Polymer
a) Monomere umfasst, die unter Bildung von Aldehydgruppen oxidiert sind, und
b) kovalent an das Antigen gebunden ist;
zur Herstellung eines Impfstoffs zur intranasalen Verabreichung zur Erzeugung einer Immunantwort auf das Antigen, umfassend eine sekretorische Immunantwort.

2. Verwendung gemäß Anspruch 1, wobei die Immunantwort eine humorale Immunantwort umfasst.

3. Verwendung gemäß Anspruch 1 oder 2, wobei die Immunantwort eine IgA-Antwort umfasst.

4. Verwendung gemäß einem beliebigen der Ansprüche 1 bis 3, wobei die Immunantwort erhöhte Antikörperlevel an Stellen in der Mukosa umfasst.

5. Verwendung gemäß einem beliebigen der Ansprüche 1 bis 4, wobei die Immunantwort erhöhte Antikörperlevel im Serum umfasst.

6. Verwendung gemäß einem beliebigen der Ansprüche 1 bis 5, wobei die Immunantwort die Stimulation von Mediatoren von Th1- und/oder Th2-Zellimmunität umfasst.

7. Verwendung gemäß einem beliebigen der Ansprüche 1 bis 6, wobei die IgE-Produktion reduziert oder unverändert ist.

8. Verwendung gemäß einem beliebigen der Ansprüche 1 bis 7, wobei das Antigen ausgewählt ist aus der Gruppe, bestehend aus den Folgenden oder immunogenen Teilen davon: Pollen, Allergene, Bakterien, Viren, Hefen, Pilzen, Protozoen oder anderen Mikroorganismen, umfassend Pathogene von Menschen, Tieren und Pflanzen.

9. Verwendung gemäß Anspruch 8, wobei das Antigen ausgewählt ist aus der Gruppe, bestehend aus Influenza-Virus, Influenza-Haemagglutinin, *Porphyromona gingivalis*, Proteinase- und Adhesinepitope von *Porphyromona gingivalis*, *Helicobacter pylori, Helicobacter* pylori-Urease, Rotavirus, rekombinantes VP5-Protein von Rotavirus, HIV, HIV-gp120, RSV, Oberflächenproteinen von RSV und Ovalbuminpeptide und *Listeria monocytogenes*, *Mycobacterium tuberculosis, Mycobacterium avium*, BCG, Influenza Nucleoprotein, Respiratory-Syncyticial-Virus (RSV) F- oder G-Proteine, *Candida albicans* und *Chlamydia trachomatis* oder äußere Membranproteine davon, *Neisseria meningitidis* Klasse 1 äußeres Protein, Herpes simplex Virus Typ I Glycoprotein G oder gp D oder CP27, menschliches Papilloma-Virus, Murray-Valley-Encephalitis-Virus E-Glycoprotein, antigene Teile davon und Derivate davon.

10. Verwendung gemäß Anspruch 8 oder 9, wobei das Antigen ausgewählt ist aus der Gruppe, bestehend aus HIV-Antigenen, Malaria-Antigenen, Antigenen von Mikroorganismen, welche Geschlechtskrankheiten, herkömmliche Erkältung oder Influenza verursachen, und Antigenen, die Asthma induzieren.

## Revendications

1. Utilisation d'une composition comprenant un antigène et un adjuvant à base de polymère de glucide comprenant du mannose, où ledit polymère
a) comprend des monomères de mannose oxydés pour produire des groupes aldéhydes, et
b) est lié de manière covalente à l'antigène ;
pour la préparation d'un vaccin destiné à une administration intranasale pour générer une réponse immunitaire contre l'antigène comprenant une réponse immunitaire sécrétoire.

2. Utilisation selon la revendication 1, où la réponse immunitaire comprend une réponse immunitaire humorale.

3. Utilisation selon la revendication 1 ou la revendication 2, où la réponse immunitaire comprend une réponse d'IgA.

4. Utilisation selon l'une quelconque des revendications 1 à 3, où la réponse immunitaire comprend une augmentation des taux d'anticorps au niveau de sites mucosaux.

5. Utilisation selon l'une quelconque des revendications 1 à 4, où la réponse immunitaire comprend une augmentation des taux d'anticorps dans le sérum.

6. Utilisation selon l'une quelconque des revendications 1 à 5, où la réponse immunitaire comprend une stimulation de médiateurs de l'immunité cellulaire Th1 et/ou Th2.

7. Utilisation selon l'une quelconque des revendications 1 à 6, où la production d'IgE est réduite ou inchangée.

8. Utilisation selon l'une quelconque des revendications 1 à 7, où l'antigène est choisi dans le groupe constitué des suivants ou de parties immunogéniques de ceux-ci : pollens, allergènes, bactéries, virus, levures, champignons, protozoaires ou d'autres micro-organismes y compris des agents pathogènes des êtres humains, des animaux et des végétaux.

9. Utilisation selon la revendication 8, où l'antigène est choisi dans le groupe constitué par le virus de la grippe, l'hémagglutinine de la grippe, *Porphyromona gingivalis*, des épitopes de protéinases et d'adhésines de *Porphyromona gingivalis*, *Helicobacter pylori*, l'uréase de *Helicobacter pylori*, des rotavirus, la protéine VP5 recombinante de rotavirus, le VIH, la gp120 du VIH, le RSV, des protéines de surface du RSV et des peptides de l'ovalbumine et *Listeria monocytogenes*, *Mycobacterium tuberculosis, Mycobacterium avium*, le BCG, la nucléoprotéine de la grippe, des protéines F ou G du virus respiratoire syncytial (RSV), *Candida albicans* et *Chlamydia trachomatis* ou la protéine de la membrane externe de celles-ci, la protéine externe de classe 1 de *Neisseria meningitidis,* la glycoprotéine G de type 1 ou la gp D ou CP27 du virus Herpes simplex, le virus du papillome humain, la glycoprotéine E du virus de l'encéphalite de la vallée de la Murray, des parties antigéniques de ceux-ci et des dérivés de ceux-ci.

10. Utilisation selon la revendication 8 ou la revendication 9, où l'antigène est choisi dans le groupe constitué d'antigènes du VIH, d'antigènes du paludisme, d'antigènes provenant de micro-organismes qui provoquent des maladies vénériennes, un rhume banal ou la grippe et d'antigènes qui induisent l'asthme.
